# EUROPEAN PATENT APPLICATION

(11) **EP 4 617 275 A1**
(43) Date of publication of application: **17.09.2025**
(21) Application number: 23887808.6
(22) Date of filing: 28.10.2023
(51) Int. Cl.: C07D 493/08, A01N 43/02

(54) **OXABICYCLOALKANE COMPOUNDS, PREPARATION METHOD THEREFOR, AND HERBICIDAL COMPOSITION AND USE THEREOF**

(30) Priority: 09.11.2022 CN 202211395248; 12.12.2022 CN 202211597464
(71) Applicant: Qingdao KingAgroot Chemical Compound Co., Ltd., Qingdao, Shandong 266000 (CN)
(72) Inventor: LIAN, Lei, Qingdao, Shandong 266000 (CN); HUA, Rongbao, Qingdao, Shandong 266000 (CN); PENG, Xuegang, Qingdao, Shandong 266000 (CN)
(74) Representative: De Vries & Metman
(86) International application number: PCT/CN2023/127411
(87) International publication number: WO 2024/099118

(57) **Abstract**

The present invention belongs to the technical field of pesticides and particularly relates to an oxabicycloalkane compound, as well as a preparation method, a herbicidal composition and an application thereof. The compound is as shown in the general formula I: 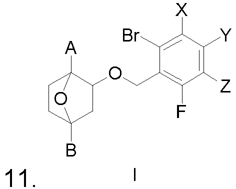 wherein, A and B each independently represent alkyl; X, Y, and Z each independently represent hydrogen, nitro, halogen, etc. The compound has superior crop safety while maintaining comparable or even better herbicidal activity and is able to build better selectivity in crops.

## Description

### Technical Field

The present invention belongs to the technical field of pesticides and particularly relates to an oxabicycloalkane compound, as well as a preparation method, a herbicidal composition and an application thereof.

### Background Art

Weed control is one of the most important links in the course of achieving high-efficiency agriculture. Although various herbicides are available in the market, for example, patents CN1045786A and the like disclose the method of a class of oxabicycloalkane compound herbicides for controlling harmful plant growth in paddy fields, the herbicidal properties against harmful plants and the selectivity to crops of these known compounds are not completely satisfactory. Moreover, due to the continuous expansion of the market, issues such as resistance of weeds, service life of drugs and economy of drugs, as well as people's increasing attention to the environment, scientists are requested to continuously study and further develop new efficient, safe and economical herbicide varieties with different modes of action.

### Description of the Invention

In order to solve the above-mentioned problems existing in the prior art, the invention provides an oxabicycloalkane compound, as well as a preparation method, a herbicidal composition and an application thereof. The present compound has superior crop safety while maintaining comparable or even better herbicidal activity and is able to build better selectivity in crops.

The technical scheme adopted in the present invention is as follows:
An oxabicycloalkane compound is as shown in the general formula I: 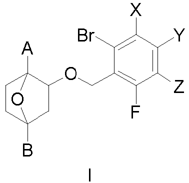 wherein, A and B each independently represent alkyl;
X, Y, and Z each independently represent hydrogen, nitro, halogen, cyano, formyl, thiocyanato, sulfhydryl, OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -alkyl-C(S)R¹, -alkyl-C(S)OR¹, -alkyl-C(O)SR¹, -alkyl-C(S)SR¹, -alkyl-SC(O)R¹, -alkyl-OC(S)R¹, - alkyl-SC(S)R¹, -O-alkyl-NR⁴R⁵, -S-alkyl-NR⁴R⁵, -alkyl-O-alkyl-NR⁴R⁵, -alkyl-S-alkyl-NR⁴R⁵, -alkyl-(C=S)ₙ-NR⁴R⁵, -NH-alkyl-NR⁴R⁵, -alkyl-OR¹, -alkyl-COR¹, - alkyl-CO₂R¹, -alkyl-OCOR¹, -alkyl-NR³COR¹, -alkyl-SO₂OR¹, -alkyl-NR³SO₂R², -alkyl-OSO₂R², -alkyl-S(O)ₘR², -alkyl-CONR⁴R⁵, -alkyl-SO₂NR⁴R⁵, NR⁴R⁵, 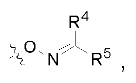 P(O)(OR⁶)₂, CH₂P(O)(OR⁶)₂, -alkyl-CN, alkyl, alkenyl, alkynyl, cycloalkyl, cycloalkylalkyl, cycloalkenyl, cycloalkenylalkyl, heterocyclyl, heterocyclylalkyl, aryl, or arylalkyl; wherein, the "alkyl", "alkenyl" or "alkynyl" are each independently unsubstituted or substituted by halogen; the "cycloalkyl", "cycloalkylalkyl", "cycloalkenyl", "cycloalkenylalkyl", "heterocyclyl", "heterocyclylalkyl", "aryl" or "arylalkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, cycloalkyl substituted by alkyl, -OR¹⁰, -SR¹⁰, -(CO)OR¹⁰, -(SO₂)R¹⁰, -N(R¹⁰)₂ or -O-alkyl-(CO)OR¹⁰, or two adjacent carbon atoms on the ring form a fused ring with -OCH₂CH₂- or -OCH₂O- that is unsubstituted or substituted by halogen;
R¹, R³, R⁴, and R⁵ each independently represent hydrogen, aryl, arylalkyl, heterocyclyl, heterocyclylalkyl, alkyl, haloalkyl, alkenyl, haloalkenyl, alkynyl, haloalkynyl, cycloalkyl, halocycloalkyl, alkoxyalkyl, cycloalkylalkyl; wherein the latter 10 groups are substituted by s substituents selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and alkoxyalkoxycarbonyl;
R² is aryl, arylalkyl, heterocyclyl, heterocyclylalkyl, alkyl, alkenyl, alkynyl, cycloalkyl, cycloalkylalkyl; wherein the latter 5 groups are substituted by s groups selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ and alkoxyalkoxycarbonyl;
R⁶ is methyl or ethyl;
R⁷ and R⁸ each independently represent hydrogen, alkyl, alkenyl, or alkynyl;
R⁹ is alkyl, alkenyl, or alkynyl;
R¹⁰ independently represents hydrogen, alkyl, haloalkyl, phenyl or phenyl substituted by at least one group selected from halogen, cyano, nitro, alkyl, haloalkyl, alkoxycarbonyl, alkylthio, alkylsulfonyl, alkoxy, or haloalkoxy;
m represents 0, 1, or 2; n represents 0 or 1; s represents 0, 1, 2, 3, 4, or 5.

In one specific embodiment. A and B each independently represent C1-C8 alkyl;
X, Y, and Z each independently represent hydrogen, nitro, halogen, cyano, formyl, cyanothio, sulfhydryl, OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C8 alkyl)-C(S)R¹, -(C1-C8 alkyl)-C(S)OR¹, -(C1-C8 alkyl)-C(O)SR¹, -(C1-C8 alkyl)-C(S)SR¹, -(C1-C8 alkyl)-SC(O)R¹, -(C1-C8 alkyl)-OC(S)R¹, -(C1-C8 alkyl)-SC(S)R¹, -O-(C1-C8 alkyl)-NR⁴R⁵, -S-(C1-C8 alkyl)-NR⁴R⁵, -(C1-C8 alkyl)-O-(C1-C8 alkyl)-NR⁴R⁵, - (C1-C8 alkyl)-S-(C1-C8 alkyl)-NR⁴R⁵, -(C1-C8 alkyl)-(C=S)ₙ-NR⁴R⁵, -NH-(C1-C8 alkyl)-NR⁴R⁵, -(C1-C8 alkyl)-OR¹, -(C1-C8 alkyl)-COR¹, -(C1-C8 alkyl)-CO₂R¹, -(C1-C8 alkyl)-OCOR¹, -(C1-C8 alkyl)-NR³COR¹, -(C1-C8 alkyl)-SO₂OR¹, -(C1-C8 alkyl)-NR³SO₂R², -(C1-C8 alkyl)-OSO₂R², -(C1-C8 alkyl)-S(O)ₘR², -(C1-C8 alkyl)-CONR⁴R⁵, -(C1-C8 alkyl)-SO₂NR⁴R⁵, NR⁴R⁵, 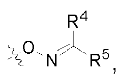 P(O)(OR⁶)₂, CH₂P(O)(OR⁶)₂, -(C1-C8 alkyl)-CN, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl, or aryl C1-C8 alkyl; wherein, the "C1-C8 alkyl", "C2-C8 alkenyl" or "C2-C8 alkynyl" are each independently unsubstituted or substituted by halogen; the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" or "aryl C1-C8 alkyl" are each independently unsubstituted or substituted by at least one group selected form oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C3-C8 cycloalkyl substituted by C1-C8 alkyl, -OR¹⁰, -SR¹⁰, -(CO)OR¹⁰, -(SO₂)R¹⁰, -N(R¹⁰)₂, or - O-(C1-C8 alkyl)-(CO)OR¹⁰, or two adjacent carbon atoms on the ring form a fused ring with -OCH₂CH₂- or -OCH₂O- that is unsubstituted or substituted by halogen;
R¹, R³, R⁴, and R⁵ each independently represent hydrogen, aryl, aryl C1-C8 alkyl, heterocyclyl, heterocyclyl C1-C8 alkyl, C1-C8 alkyl, halo C1-C8 alkyl, C2-C8 alkenyl, halo C2-C8 alkenyl, C2-C8 alkynyl, halo C2-C8 alkynyl, C3-C8 cycloalkyl, halo C3-C8 cycloalkyl, C1-C8 alkoxy C1-C8 alkyl, C3-C8 cycloalkyl C1-C8 alkyl; wherein the latter 10 groups are substituted by s substituents selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C8 alkoxy C1-C8 alkoxycarbonyl;
R² is aryl, aryl C1-C8 alkyl, heterocyclyl, heterocyclyl C1-C8 alkyl, C1-C8 alkyl, C2-C8 alkenyl, C2-C8 alkynyl, C3-C8 cycloalkyl, C3-C8 cycloalkyl C1-C8 alkyl; wherein the latter 5 groups are substituted by s groups selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C8 alkoxy C1-C8 alkoxycarbonyl;
R⁶ is methyl or ethyl;
R⁷ and R⁸ each independently represent hydrogen, C1-C8 alkyl, C2-C8 alkenyl, or C2-C8 alkynyl;
R⁹ is C1-C8 alkyl, C2-C8 alkenyl, or C2-C8 alkynyl;
R¹⁰ independently represents hydrogen, C1-C8 alkyl, halo C1-C8 alkyl, phenyl, or phenyl substituted by at least one group selected from halogen, cyano, nitro, C1-C8 alkyl, halo C1-C8 alkyl, C1-C8 alkoxycarbonyl, C1-C8 alkylthio, C1-C8 alkylsulfonyl, C1-C8 alkoxy, or halo C1-C8 alkoxy;
m represents 0, 1, or 2; n represents 0 or 1; s represents 0, 1, 2, 3, 4, or 5.

In another specific embodiment, A and B each independently represent C1-C6 alkyl; preferably, A represents methyl or ethyl; B represents methyl, ethyl, or isopropyl;
X, Y, and Z each independently represent hydrogen, nitro, halogen, cyano, formyl, thiocyanato, sulfhydryl, OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C6 alkyl)-C(S)R¹, -(C1-C6 alkyl)-C(S)OR¹, -(C1-C6 alkyl)-C(O)SR¹, -(C1-C6 alkyl)-C(S)SR¹, -(C1-C6 alkyl)-SC(O)R¹, -(C1-C6 alkyl)-OC(S)R¹, -(C1-C6 alkyl)-SC(S)R¹, -O-(C1-C6 alkyl)-NR⁴R⁵, -S-(C1-C6 alkyl)-NR⁴R⁵, -(C1-C6 alkyl)-O-(C1-C6 alkyl)-NR⁴R⁵, - (C1-C6 alkyl)-S-(C1-C6 alkyl)-NR⁴R⁵, -(C1-C6 alkyl)-(C=S)ₙ-NR⁴R⁵, -NH-(C1-C6 alkyl)-NR⁴R⁵, -(C1-C6 alkyl)-OR¹, -(C1-C6 alkyl)-COR¹, -(C1-C6 alkyl)-CO₂R¹, -(C1-C6 alkyl)-OCOR¹, -(C1-C6 alkyl)-NR³COR¹, -(C1-C6 alkyl)-SO₂OR¹, -(C1-C6 alkyl)-NR³SO₂R², -(C1-C6 alkyl)-OSO₂R², -(C1-C6 alkyl)-S(O)ₘR², -(C1-C6 alkyl)-CONR⁴R⁵, -(C1-C6 alkyl)-SO₂NR⁴R⁵, NR⁴R⁵, 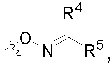 P(O)(OR⁶)₂, CH₂P(O)(OR⁶)₂, -(C1-C6 alkyl)-CN, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl or aryl C1-C6 alkyl; wherein, the "C1-C6 alkyl", "C2-C6 alkenyl" or "C2-C6 alkynyl" are each independently unsubstituted or substituted by halogen; the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" or "aryl C1-C6 alkyl" are each independently unsubstituted or substituted by at least one group selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C3-C6 cycloalkyl substituted by C1-C6 alkyl, -OR¹⁰, -SR¹⁰, -(CO)OR¹⁰, -(SO₂)R¹⁰, -N(R¹⁰)₂ or -O-(C1-C6 alkyl)-(CO)OR¹⁰, or two adjacent carbon atoms on the ring form a fused ring with -OCH₂CH₂- or -OCH₂O- that is unsubstituted or substituted by halogen;
R¹, R³, R⁴, and R⁵ each independently represent hydrogen, aryl, aryl C1-C6 alkyl, heterocyclyl, heterocyclyl C1-C6 alkyl, C1-C6 alkyl, halo C1-C6 alkyl, C2-C6 alkenyl, halo C2-C6 alkenyl, C2-C6 alkynyl, halo C2-C6 alkynyl, C3-C6 cycloalkyl, halo C3-C6 cycloalkyl, C1-C6 alkoxy C1-C6 alkyl, C3-C6 cycloalkyl C1-C6 alkyl; wherein the latter 10 groups are substituted by s substituents selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C6 alkoxy C1-C6 alkoxycarbonyl;
R² is aryl, aryl C1-C6 alkyl, heterocyclyl, heterocyclyl C1-C6 alkyl, C1-C6 alkyl, C2-C6 alkenyl, C2-C6 alkynyl, C3-C6 cycloalkyl, C3-C6 cycloalkyl C1-C6 alkyl; wherein the latter 5 groups are substituted by s groups selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C6 alkoxy C1-C6 alkoxycarbonyl;
R⁶ is methyl or ethyl;
R⁷ and R⁸ each independently represent hydrogen, C1-C6 alkyl, C2-C6 alkenyl, or C2-C6 alkynyl;
R⁹ is C1-C6 alkyl, C2-C6 alkenyl, or C2-C6 alkynyl;
R¹⁰ independently represents hydrogen, C1-C6 alkyl, halo C1-C6 alkyl, phenyl, or phenyl substituted by at least one group selected from halogen, cyano, nitro, C1-C6 alkyl, halo C1-C6 alkyl, C1-C6 alkoxycarbonyl, C1-C6 alkylthio, C1-C6 alkylsulfonyl, C1-C6 alkoxy, or halo C1-C6 alkoxy;
m represents 0, 1, or 2; n represents 0 or 1; s represents 0, 1, 2, 3, 4, or 5.

In the definitions of the compounds represented by the above-mentioned general formulas and all of the following structural formulas, the technical terms used, whether used alone or used in compound words, represent the following substituent groups: an alkyl group which has more than two carbon atoms and may be linear or branched. For example, the alkyl in the compound word "-alkyl-C(S)R¹" may be -CH₂-, -CH₂CH₂-, -CH(CH₃)-, -C(CH₃)₂-, etc. An alkyl group is, for example, C1 alkyl: methyl; C2 alkyl: ethyl; C3 alkyl: propyl such as n-propyl or isopropyl; C4 alkyl: butyl such as n-butyl, isobutyl, tert-butyl, or 2-butyl; C5 alkyl: pentyl such as n-pentyl; C6 alkyl: hexyl such as n-hexyl, isohexyl, and 1,3-dimethylbutyl. Similarly, an alkenyl is, for example, vinyl, allyl, 1-methylprop-2-en-1-yl, 2-methylprop-2-en-1-yl, but-2-en-1-yl, but-3-en-1-yl, 1-methylbut-3-en-1-yl, and 1-methylbut-2-en-1-yl. An alkynyl is, for example, ethynyl, propargyl, but-2-yn-1-yl, but-3-yn-1-yl, and 1-methylbut-3-yn-1-yl. A multiple bond may be placed at any position of each unsaturated group. A cycloalkyl is a saturated carbocyclic ring system having, for example, three to six carbon atoms such as cyclopropyl, cyclobutyl, cyclopentyl, or cyclohexyl. Similarly, a cycloalkenyl is a monocyclic alkenyl having, for example, three to six carbon ring members such as cyclopropenyl, cyclobutenyl, cyclopentenyl, and cyclohexenyl, wherein a double bond can be at any position. A halogen is fluorine, chlorine, bromine, or iodine.

Unless otherwise specified, the "aryl" of the present invention includes, but is not limited to, phenyl, naphthyl, 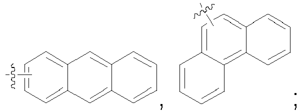 the "heterocyclyl" not only includes, but is not limited to, saturated or unsaturated non-aromatic cyclic groups 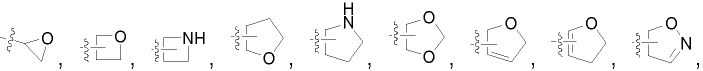 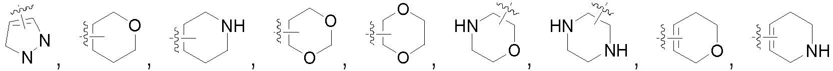 or 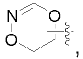 etc., but also includes, but is not limited to, heteroaryl, that is an aromatic cyclic group having, for example, 3 to 6 ring atoms and also a benzo ring optionally fused with. One to four (for example, 1, 2, 3, or 4) heteroatoms of the ring atoms are selected from oxygen, nitrogen, and sulfur, for example

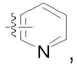

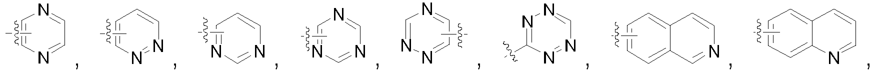

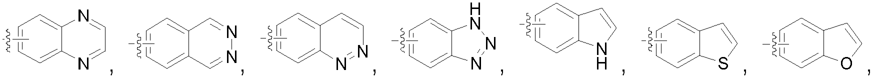

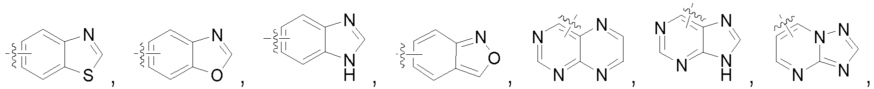

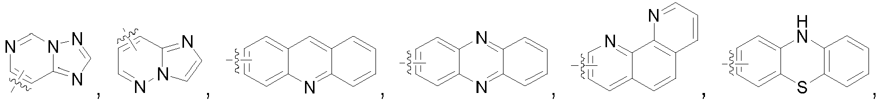

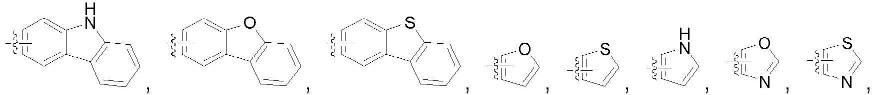

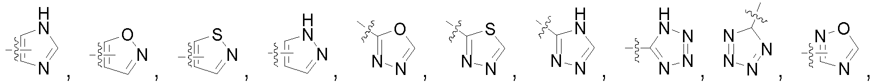

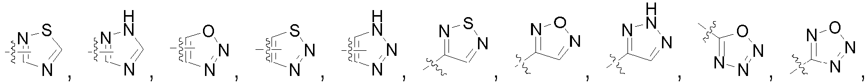

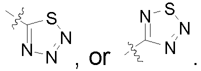

If one group is substituted by a group, which should be understood to mean that the group is substituted by one or more same or different groups selected from the mentioned groups. In addition, the same or different substitution characters contained in the same or different substituent groups are independently selected, which may be the same or different. This is also applicable to ring systems formed by different atoms and units. Meanwhile, the scope of the claims will exclude those compounds chemically unstable under standard conditions which are known to a person skilled in the art.

In addition, unless specifically defined, the term "substituted by at least one group" herein refers to being substituted by, for example, 1, 2, 3, 4, or 5 groups; a group (including heterocyclyl, aryl, etc.) without being specified a linking site may be linked at any site, including a site connected to C or N; if it is substituted, the substituent group may also substitute at any site as long as the valence bond theory is complied with. For example, a heteroaryl 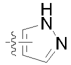 substituted by 1 methyl may represent 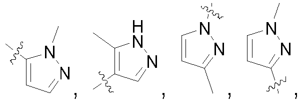 etc.

According to the nature of the substituent groups and the manner in which they are connected, the compound of the general formula I and derivatives thereof may exist as stereoisomers. Stereoisomers may be obtained by conventional separation methods, for example by chromatographic separation, from mixtures produced in preparation. Stereoisomers may also be selectively prepared by employing stereoselective reactions and using optically active starting materials and/or auxiliaries. The present invention further relates to all stereoisomers which are included in the general formula I but not specifically defined, and mixtures thereof.

The present invention also provides a preparation method of the oxabicycloalkane compound, comprising the following steps:
subjecting the compound shown in the general formula II and the compound shown in the general formula III to substitution reaction to produce the compound of the general formula I; the chemical reaction equation is as follows: 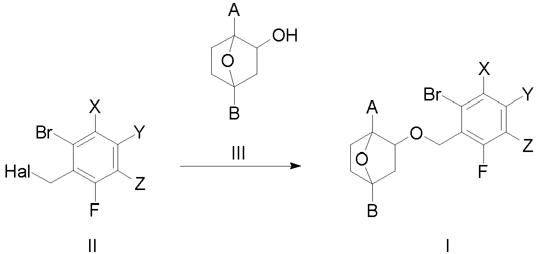
wherein, Hal represents halogen, and the definitions of other substituent groups A, B, X, Y, and Z are as shown above;
preferably, the reaction is carried out in the presence of NaH or anhydrous carbonate (e.g., potassium salt, sodium salt) and a solvent; more preferably, the solvent is selected from at least one of DMF, dimethylacetamide, THF, acetonitrile, dichloroethane, DMSO, dioxane, dichloromethane, or ethyl acetate.

The compound of the present invention may be prepared by referring to the related methods described in patents CN1045786A, etc.

The present invention also provides a herbicidal composition, which comprises (i) a herbicidally effective amount of at least one of the oxabicycloalkane compounds;
optionally, the herbicidal composition further comprises (ii) a herbicidally effective amount of one or more additional herbicides and/or safeners;
preferably, the herbicidal composition further comprises (iii) an agrochemically acceptable formulation auxiliary.

The present invention also provides a method for controlling undesirable plants, comprising applying a herbicidally effective amount of at least one of the oxabicycloalkane compounds or the herbicidal composition to a plant, or in its area, or to soil or water to control the emergence or growth of undesirable plants.

Preferably, the undesirable plants include herbicide-resistant or -tolerant weed species.

The present invention also provides a use of at least one of the oxabicycloalkane compounds or the herbicidal composition for controlling undesirable plants.

Preferably, the oxabicycloalkane compound is used to control weeds among useful crops.

More preferably, the useful crops include transgenic crops or crops treated by genome editing techniques, and the weeds include herbicide-resistant or - tolerant weed species.

The compounds of Formula I of the present invention have outstanding herbicidal activity against a broad spectrum of monocotyledonous and dicotyledonous harmful plants of economic importance. The active substances of the present invention also act efficiently on perennial weeds which grow from root stocks, rhizomes, or other perennial organs and are difficult to control. In this context, it is generally immaterial whether the substances are applied before sowing, before emergence or after emergence. Representative examples of monocotyledonous and dicotyledonous weed florae which can be controlled by the compounds of the present invention may be specifically mentioned, without limiting to certain species. Examples of weed species on which the active substances act efficiently include the monocotyledons such as annual *Avena, Lolium, Alopecurus, Phalaris, Echinochloa, Digitaria, Setaria,* and *Cyperus* as well as perennial *Agropyron, Cynodon, Imperata* and *Sorghum,* and perennial *Cyperus.*

In the case of the dicotyledonous weed species, the spectrum of action extends to species such as annual *Galium, Viola, Veronica, Lamium, Stellaria, Amaranthus, Sinapis, Ipomoea, Sida, Matricaria,* and *Abutilon* as well as perennial weeds *Convolvulus, Cirsium, Rumex,* and *Artemisia.* The active substances of the present invention also perform outstanding control of harmful plants such as *Echinochloa, Sagittaria, Alisma, Eleocharis, Scirpus,* and *Cyperus* under the specific conditions of rice growing. If the compounds of the present invention are applied to soil surface prior to germination, the weed seedlings are either prevented completely from emerging, or the weeds stop growing when reaching the cotyledon stage and eventually died completely after three to four weeks. In particular, the compounds of the present invention exhibit excellent activity against *Apera spica venti, Matsumurella chinense, Fallopia convolvulus, Stellaria media, Veronica hederifolia, Veronica persica, Viola tricolor, Amaranthus, Galium,* and *Kochia.*

Although the compounds of the present invention have excellent herbicidal activity against monocotyledonous and dicotyledonous weeds, crop plants of economic importance such as peanut, wheat, barley, rye, rice, corn, sugarbeet, cotton, and soya are not damaged at all, or only to a negligible extent. **In** particular, they have excellent compatibility with cereals such as wheat, barley, and corn, in particular wheat. For these reasons, the compounds of the present invention are highly suitable for selectively controlling undesired plant growth in plantings for agricultural or ornamental use.

Owing to their herbicidal properties, these active substances may be employed for controlling harmful plants in plantings of genetically engineered plants that are known or to be introduced. Transgenic plants usually have advantageous traits, for example, resistance to certain pesticides, in particular to certain herbicides; resistance to plant diseases or pathogenic microorganisms of plant diseases such as certain insects or microorganisms including fungi, bacteria, or viruses. Other particular traits relate to the following conditions of the product, for example, quantity, quality, storage stability, composition, and special ingredients. Thus, it is known that the obtained transgenic plant product has an increased starch content, or modified starch quality, or a different fatty acid composition.

The compounds of Formula I of the present invention or salts thereof are preferably used in transgenic crops and ornamental plants of economic importance, for example, cereals such as wheat, barley, rye, oats, millet, rice, manioc and corn, or else in plantings of sugarbeet, cotton, soya, rapeseed, potato, tomato, pea, and other vegetable plants. The compounds of Formula I are preferably used as herbicides in plantings of useful plants which are resistant, or have been made resistant by genetic engineering, against the toxic effects of herbicides.

Conventional ways for breeding plants which have modified traits compared to known plants include, for example, conventional breeding methods and breeding of mutant strains. In other words, novel plants having improved traits may be generated with the aid of genetic engineering methods (see, e.g., EP-0221044 A, EP-0131624 A). For example, several methods have been described:
- changing crop plants using genetic engineering to modify the starch synthesis in the plants (e.g., WO 92/11376, WO 92/14827, WO 91/19806);
- transgenic crop plants which are resistant to certain herbicides, including glufosinate-P herbicides (e.g., EP-0242236 A, EP-0242246 A), or glyphosate herbicides(WO 92/00377), or sulfonylurea herbicides (EP-0257993 A, US-5013659 A);
- transgenic crop plants, for example cotton, able to produce *Bacillus thuringiensis* toxins (*Bt* toxins) which impart resistance to certain pests that invade the plants (EP-0142924 A, EP-0193259 A);
- transgenic crop plants having a modified fatty acid composition (WO 91/13972).

Numerous molecular biology techniques which allow the preparation of transgenic plants having modified traits are known (see, e.g., Sambrook et al., 1989, Molecular Cloning, A Laboratory Manual, 2nd ed. Cold Spring Harbor Laboratory Press, Cold Spring Harbor, N.Y.; or Winnacker, "Gene und Klone" [Genes and Clones], VCH Weinheim, 2nd edition, 1996; or Christou, "Trends in Plant Science", 1 (1996) 423-431). In order to carry out genetic engineering manipulations, it is possible to introduce nucleic acid molecules into plasmids, allowing a mutagenesis or a change in the sequence to occur by recombination of DNA sequences. Using the above-mentioned standard processes, it is possible to, for example, substitute bases, remove partial sequences, or add natural or synthetic sequences. To link the DNA fragments with each other, it is possible to attach adaptors or linkers to the fragments.

Plant cells having gene products of reduced activity may be prepared by the following methods, for example, by expressing at least one appropriate antisense-RNA and one sense-RNA to achieve a cosuppression effect, or by expressing at least one appropriately constructed ribozyme which specifically cleaves transcripts of the above-mentioned gene products.

To this end, it is possible to employ both DNA molecules which comprise the entire coding sequence of a gene product including any flanking sequences that may be present, and DNA molecules which comprise only parts of the coding sequence that must be long enough to cause an antisense effect in the cells. It is also possible to use sequences which have a high degree of homology but are not entirely identical to the coding sequences of a gene product.

When nucleic acid molecules are expressed in plants, the synthesized protein may be localized in any desired compartment of the plant cells. However, to achieve localization in a certain compartment, it is possible, for example, to link the coding region with DNA sequences to ensure localization in a certain compartment. Such sequences are known to a person skilled in the art (see, e.g., Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Transgenic plant cells can be recombined onto whole plants using known techniques. Transgenic plants may be of any desired plant species, i.e., monocotyledonous and dicotyledonous plants. In this manner, it is possible to obtain transgenic plants having modified traits by overexpression, suppression or inhibition of homologous (= natural) genes or gene sequences, or by expression of heterologous (= foreign) genes or gene sequences.

When using the active substances of the present invention in transgenic crops, in addition to the inhibitory effect against harmful plants which can be observed in other crops, there are frequently special effects on the corresponding transgenic crops, for example, improving or broadening the spectrum of weeds which can be controlled, modifying the application amount during application, good combination of the drug resistance of preferred transgenic crops and herbicide performance, as well as effects on the growth and the yield of transgenic crop plants. The present invention therefore also provides a use of the compounds as herbicides for controlling harmful plants among transgenic crop plants.

In addition, the compounds of the present invention are able to significantly regulate the growth of crop plants. These compounds are employed for the targeted control of plant constituents and for facilitating harvesting, for example by provoking desiccation and stunted growth, by engaging in plant metabolism in a regulating manner. Furthermore, they are also suitable for regulating and inhibiting undesirable plant growth without destroying the growth of crop plants. Inhibition of plant growth plays an important role in many monocotyledonous and dicotyledonous crops because it can reduce or completely prevent lodging hereby.

The compounds of the present invention may be applied in customary formulations in the form of wettable powders, emulsifiable concentrates, sprayable solutions, dusts or granules. The present invention therefore also provides herbicidal compositions comprising the compounds of Formula I. The compounds of Formula I may be formulated in various ways depending on the prevailing physical parameters in biology and/or chemistry. Examples of suitable formulation options are: wettable powders (WP), water-soluble powders (SP), water-soluble concentrates, emulsifiable concentrates (EC), emulsions (EW) such as oil-in-water and water-in-oil emulsions, sprayable solutions, suspension concentrates (SC), oil dispersions (OD), oil- or waterbased dispersions, oil-miscible solutions, dust powders (DP), capsule suspensions (CS), seed-dressing compositions, granules for broadcasting and soil application, spray granules, coating granules and adsorption granules, water-dispersible granules (WG), water-soluble granules (SG), ULV formulations, microcapsules, and waxes. These individual formulation types are known and are described in the following literature, for example, Winnacker-Küchler, "Chemische Technologie" [Chemical Technology], Volume 7, C. Hauser Verlag Munich, 4th. Edition, 1986; Wade van Valkenburg, "Pesticide Formulations", Marcel Dekker, N.Y, 1973; K. Martens, "Spray Drying" Handbook, 3rd Ed. 1979, G. Goodwin Ltd. London.

Necessary formulation auxiliaries such as inert materials, surfactants, solvents and other additives are likewise known, and are described in the following documents, for example, Watkins, "Handbook of Insecticide Dust Diluents and Carriers", 2nd Ed., Dorland Books, Caldwell N.J.; H. v. Olphen, "An Introduction to Clay Colloid Chemistry", 2nd Ed., J. Wiley & Sons, N.Y.; C. Marsden, "Solvents Guide", 2nd Ed., Interscience, N.Y. 1963; "McCutcheon's Detergents and Emulsifiers Annual", MC Publ. Corp., Ridgewood N.J.; Sisley and Wood, "Encyclopedia of Surface Active Agents", Chem. Publ. Co. Inc., N.Y, 1964; Schönfeldt, "Grenzflüchenaktive Äthylenoxidaddkte" [Surface-active ethylene oxide adducts], Wiss. Verlagagesell., Stuttgart, 1976; Winnacker-Küchler, "Chemische Technologie" [Chemical Technology], Volume 7, C. Hauser Verlag Munich, 4th Edition, 1986.

Wettable powders are uniformly dispersible in water which contain, in addition to the active substance, a diluent or inert substance, ionic and/or nonionic surfactants (wetting agents, dispersants), for example polyethoxylated alkyl phenols, polyethoxylated fatty alcohols, polyethoxylated fatty amines, fatty alcohol polyglycol ether sulfates, alkane sulfonates, alkylphenyl sulfonates, sodium lignosulfonates, sodium 2,2'-dinaphthylmethane-6,6'-disulfonates, sodium dibutylnaphthalenesulfonate, or sodium methyl oleoyl taurate. To prepare the wettable powders, the herbicidally active substance is finely ground, for example, in customary apparatus such as hammer mills, fan mills, and airjet mills, and mixed with formulation auxiliaries simultaneously or sequentially.

Emulsifiable concentrates are prepared by dissolving the active substance in an organic solvent, for example butanol, cyclohexanone, dimethylformamide, xylene or aromatic compounds with relatively high boiling point or hydrocarbons or mixtures of the solvents, with the addition of one or more ionic and/or nonionic surfactants (emulsifiers). Examples of emulsifiers which can be used are calcium alkylarylsulfonates such as calcium dodecylbenzene sulfonate, or nonionic emulsifiers such as fatty acid polyglycol esters, alkylaryl polyglycol ethers, fatty alcohol polyglycol ethers, propylene oxide-ethylene oxide condensation products, alkyl polyethers, sorbitan esters such as sorbitan fatty acid esters, or polyoxyethylene sorbitan esters such as polyoxyethylene sorbitan fatty acid esters.

Dusts are obtained by grinding the active substance with finely divided solid substances, for example talc, natural clays such as kaolin, bentonite and pyrophyllite, or diatomaceous earth. Water- or oil-based suspension concentrates may be prepared by the following methods, for example, by wet milling using commercially customary bead mills, with or without the addition of the surfactants mentioned above in the case of the other formulation type.

Emulsions such as oil-in-water emulsions (EW) may be prepared by means of stirrers, colloid mills and/or static mixers using aqueous organic solvents and, if desired, the surfactants as mentioned above in the case of the other formulation type may be added.

Granules may be prepared by the following methods that either spraying the active substance onto adsorptive and granulating with inert materials, or concentrating the active substance onto the surface of carriers such as sand and kaolinite and granulating inert materials by means of adhesive binders such polyvinyl alcohol, sodium polyacrylate or mineral oils. Suitable active substances may also be granulated in the manner which is customary for the preparation of fertilizer granules, if desired fertilizers can be mixed in. Water-dispersible granules are generally prepared by the customary methods such as spray-drying, fluidized-bed granulation, disk granulation, mixing using highspeed mixers, and extrusion without solid inert materials.

For the preparation methods of granules by disk, fluidized-bed, extruder and spray, see the following processes in, for example, "Spray-Drying Handbook" 3rd ed. 1979, G. Goodwin Ltd., London; J. E. Browning, "Agglomeration", Chemical and Engineering, 1967, pages 147 ff.; "Perry's Chemical Engineer's Handbook", 5th Ed., McGraw-Hill, New York 1973, pp. 8-57. For further details on the formulation of crop protection products, see, for example G. C. Klingman, "Weed Control as a Science", John Wiley and Sons Inc., New York, 1961, pages 81-96 and J. D. Freyer, S.A. Evans, "Weed Control Handbook", 5th Ed., Blackwell Scientific Publications, Oxford, 1968, pages 101-103.

Agrochemical formulations generally contain from 0.1 to 99% by weight, in particular from 0.1 to 95%, of the active substance of Formula I. In wettable powders, the concentration of the active substance is, for example, from about 10 to 99% by weight, and the remainder to 100% by weight consists of customary formulation constituents. In emulsifiable concentrates, the concentration of the active substance may be from about 1 to 90%, preferably from 5 to 80%, by weight. Dust formulations contain from 1 to 30% by weight of the active substance, preferably from 5 to 20% by weight of the active substance in usual cases, while sprayable solutions contain from about 0.05 to 80%, preferably from 2 to 50%, by weight of the active substance. In the case of water-dispersible granules, the content of the active substance mainly depends on whether the active substance is liquid or solid and on auxiliaries, fillers, etc. that are used during granulation. In water-dispersible granules, the content of the active substance, for example, is between 1 and 95% by weight, preferably between 10 and 80% by weight.

In addition, the formulations of the active substances may comprise tackifiers, wetting agents, dispersants, emulsifiers, penetrants, preservatives, antifreeze agents, solvents, fillers, carriers, colorants, defoamers, evaporation suppressors as well as pH and viscosity modifiers which are usually customary in all cases.

Based on these formulations, it is also possible to produce mixtures with other pesticidally active substances, for example insecticides, acaricides, herbicides and fungicides, and also with safeners, fertilizers and/or plant growth regulators, in a manner of pre-mix or tank mix.

In mixed formulations or in tank mix formulations, suitable active substances which can be mixed with the compounds of Formula I of the present invention are the known substances described, for example in World Herbicide New Product Technology Handbook, China Agricultural Science and Farming Techniques Press, 2010.9 and in the literature cited here. For example, the active substances of herbicides mentioned below may be mixed with Compound 1 (note: the compounds are either named by the "common name" in accordance with the International Organization for Standardization (ISO) or by the chemical names, if appropriate together with a code number):
(1) HPPD (Hydroxyphenyl Pyruvate Dioxygenase) inhibitors: including but not limited to the following types,
   1) the triketones, e.g., sulcotrione (CAS No.: 99105-77-8); mesotrione (CAS No.: 104206-82-8); bicyclopyrone (CAS No.: 352010-68-5); tembotrione (CAS No.: 335104-84-2); tefuryltrione (CAS No.: 473278-76-1); and benzobicyclon (CAS No.: 156963-66-5);
   2) the diketonitriles, e.g., 2-cyano-3-cyclopropyl-1-(2-methylsulphonyl-4-trifluoromethylphenyl)-propane-1,3-dione (CAS No.: 143701-75-1); 2-cyano-3-cyclopropyl-1-(2- methylsulphonyl-3,4-dichlorophenyl)-propane-1,3-dione (CAS No.: 212829-55-5); and 2-cyano-1-[4-(methylsulphonyl)-2-trifluoromethylphenyl]-3-(1-methylcyclopropyl)propane-1,3- dione (CAS No.: 143659-52-3);
   3) the isoxazoles, e.g., isoxaflutole (CAS No.: 141112-29-0); isoxachlortole (CAS No.: 141112-06-3); and clomazone (CAS No.: 81777-89-1);
   4) the pyrazoles, e.g. topramezone (CAS No.: 210631-68-8); pyrasulfotole (CAS No.: 365400-11-9); pyrazoxyfen (CAS No.: 71561-11-0); pyrazolate (CAS No.: 58011-68-0); benzofenap (CAS No.: 82692-44-2); bipyrazone (CAS No.: 1622908-18-2); tolpyralate (CAS No.: 1101132-67-5); fenpyrazone (CAS No.: 1992017-55-6); cypyrafluone (CAS No.: 1855929-45-1); and tripyrasulfone (CAS No.: 1911613-97-2);
   5) the benzophenones;
   6) others: lancotrione (CAS No.: 1486617-21-3); fenquinotrione (CAS No.: 1342891-70-6); and flusulfinam (CAS No.: 2421252-30-2);
   and those mentioned in patent CN105264069A.
(2) EPSPS (Enolpyruvyl Shikimate Phosphate Synthase) inhibitors: e.g., sulphosate, glyphosate, glyphosate-isopropylammonium, and glyphosatetrimesium;
(3) PPO (Protoporphyrinogen Oxidase) inhibitors: classified into pyrimidinediones, diphenyl-ethers, phenylpyrazoles, N-phenylphthalimides, thiadiazoles, oxadiazoles, triazolinones, oxazolidinediones, and other herbicides with different chemical structures.

In an exemplary embodiment, the pyrimidinedione herbicides include, but are not limited to, butafenacil (CAS No.: 134605-64-4), saflufenacil (CAS No.: 372137-35-4), benzfendizone (CAS No.: 158755-95-4), tiafenacil (CAS No.: 1220411-29-9), [3-[2-chloro-4-fluoro-5-(1-methyl-6- trifluoromethyl-2,4-dioxo-1,2,3,4-tetrahydropyrimidin-3-yl)phenoxy]-2-pyridyloxy]acetic acid ethyl ester (Epyrifenacil, CAS No.: 353292-31-6), 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS No.: 1304113-05-0), 3-[7-chloro-5-fluoro-2-(trifluoromethyl)-1H-benzimidazol-4-yl]-1-methyl-6-(trifluoromethyl)-1H-pyrimidine-2,4-dione (CAS No.: 212754-02-4), flupropacil (CAS No.: 120890-70-2), isoxazoline-containing uracils disclosed in CN105753853A (e.g., compound 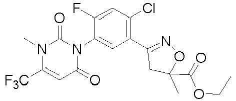 ), uracil pyridines disclosed in WO2017/202768, and uracils disclosed in WO2018/019842,
the diphenyl-ether herbicides include, but are not limited to, fomesafen (CAS No.: 72178-02-0), oxyfluorfen (CAS No.: 42874-03-3), aclonifen (CAS No.: 74070-46-5), ethoxyfen-ethyl (CAS No.: 131086-42-5), lactofen (CAS No.: 77501-63-4), chlomethoxyfen (CAS No.: 32861-85-1), chlornitrofen (CAS No.: 1836-77-7), fluoroglycofen-ethyl (CAS No.: 77501-90-7), acifluorfen or its sodium salt (CAS No.: 50594-66-6 or 62476-59-9), bifenox (CAS No.: 42576-02-3), ethoxyfen (CAS No.: 188634-90-4), fluoronitrofen (CAS No.: 13738-63-1), furyloxyfen (CAS No.: 80020-41-3), nitrofluorfen (CAS No.: 42874-01-1), and halosafen (CAS No.: 77227-69-1);
the phenylpyrazole herbicides include, but are not limited to, pyraflufenethyl (CAS No.: 129630-19-9) and fluazolate (CAS No.: 174514-07-9);
the N-phenylphthalimide herbicides include, but are not limited to, flumioxazin (CAS No.: 103361-09-7), cinidon-ethyl (CAS No.: 142891-20-1), flumipropyn (CAS No.: 84478-52-4), and flumiclorac-pentyl (CAS No.: 87546-18-7);
the thiadiazole herbicides include, but are not limited to, fluthiacet-methyl (CAS No.: 117337-19-6), fluthiacet (CAS No.: 149253-65-6), and thidiazimin (CAS No.: 123249-43-4);
the oxadiazole herbicides include, but are not limited to, oxadiargyl (CAS No.: 39807-15-3) and oxadiazon (CAS No.: 19666-30-9);
the triazolinone herbicides include, but are not limited to, carfentrazone (CAS No.: 128621-72-7), carfentrazone-ethyl (CAS No.: 128639-02-1), sulfentrazone (CAS No.: 122836-35-5), azafenidin (CAS No.: 68049-83-2), and bencarbazone (CAS No.: 173980-17-1);
the oxazolidinedione herbicides include, but are not limited to pentoxazone (CAS No.: 110956-75-7);
the other herbicides include, but are not limited to, pyraclonil (CAS No.: 158353-15-2), flufenpyr-ethyl (CAS No.: 188489-07-8), profluazol (CAS No.: 190314-43-3), trifludimoxazin (CAS No.: 1258836-72-4), N-ethyl-3-(2,6-dichloro-4-trifluoromethylphenoxy) -5-methyl-1H-pyrazole-1-carboxamide (CAS No.: 452098-92-9), N-tetrahydrofurfuryl-3-(2,6-dichloro-4-trifluoromethylphenoxy)-5-methyl-1H-pyrazole-1-carboxamide (CAS No.: 915396-43-9), N-ethyl-3-(2-chloro-6-fluoro-4-trifluoromethylphenoxy)-5-methyl-1H-pyrazole-1-carboxamide (CAS No.: 452099-05-7), N-tetrahydrofurfuryl-3-(2-chloro-6-fluoro-4-trifluoromethylphenoxy) -5-methyl-1H-pyrazole-1-carboxamide (CAS No.: 452100-03-7), 3-[7-fluoro-3-oxo-4-(prop-2-ynyl) -3,4-dihydro-2H-benzo[1,4]oxazin-6-yl]-1,5-dimethyl-6-thioxo[1,3,5]triazinane-2,4-dione (CAS No.: 451484-50-7), 2-(2,2,7-trifluoro-3-oxo-4-prop-2-ynyl-3,4-dihydro-2H-benzo[1,4]oxazin-6-yl)-4,5,6,7-tetrahydroisoindole-1,3-dione (CAS No.: 1300118-96-0), methyl(E)-4-[2-chloro-5-[4-chloro-5-(difluoromethoxy)-1H-methylpyrazol-3-yl]-4-fluorophenoxy]-3-methoxy-but-2-enoate (CAS No.: 948893-00-3), phenylpyridines disclosed in WO2016/120116, benzoxazinone derivatives disclosed in EP09163242.2, and carboxylic acid derivative-substituted imino aryl compounds disclosed in CN113105405A;
(4) ALS (Acetolactate Synthase) inhibitors include, but are not limited to, the following herbicides or mixtures thereof:
   Sulfonylureas, e.g., amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, trifloxysulfuron-sodium, triflusulfuron, triflusulfuron-methyl, and tritosulfuron;
   Imidazolinones, e.g., imazamethabenz, imazamethabenz-methyl, imazamox, imazapic, imazapyr, imazaquin, and imazethapyr;
   Triazolopyrimidine herbicides and sulfonanilides, e.g., cloransulam, cloransulam-methyl, diclosulam, flumetsulam, florasulam, metosulam, penoxsulam, pyroxsulam, pyrimisulfan, and triafamone;
   Pyrimidinyl benzoic acids, e.g. bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]benzoic acid 1-methylethyl ester (CAS No.: 420138-41-6), 4-[[[2-[(4,6-dimethoxy-2-pyrimidinyl)oxy]phenyl]methyl]amino]benzoic acid propyl ester (CAS No.: 420138-40-5), and N-(4-bromophenyl)-2-[(4,6-dimethoxy-2-pyrimidinyl)oxy]benzenemethanamine (CAS No.: 420138-01-8);
   Sulfonylaminocarbonyl-triazolinone herbicides, e.g., flucarbazone, flucarbazone-sodium, propoxycarbazone, propoxycarbazone-sodium, thiencarbazone, and thiencarbazone-methyl.

(5) ACCase (Acetyl CoA Carboxylase) inhibitors: e.g., fenthiaprop, alloxydim, alloxydim-sodium, butroxydim, clethodim, clodinafop, clodinafoppropargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-chloro-4-cyclopropyl-2'-fluoro[1,1'-biphenyl]-3-yl)-5-hydroxy-2,2,6,6-tetramethyl-2H-pyran-3(6H)-one (CAS No.: 1312337-72-6); 4-(2',4'-dichloro-4-cyclopropyl[1,1'-biphenyl]-3-yl)-5-hydroxy-2,2,6,6-tetramethyl-2H-pyran-3(6H)-one (CAS No.: 1312337-45-3); 4-(4'-chloro-4-ethyl-2'-fluoro[1,1'-biphenyl]-3-yl)-5-hydroxy-2,2,6,6-tetramethyl-2H-pyran-3(6H)-one (CAS No.: 1033757-93-5); 4-(2',4'-dichloro-4-ethyl[1,1'-biphenyl]-3-yl)-2,2,6,6-tetramethyl-2H-pyran-3,5(4H,6H)-dione (CAS No.: 1312340-84-3); 5-(acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS No.: 1312337-48-6); 5-(acetyloxy)-4-(2',4'-dichloro-4-cyclopropyl-[1,1'-biphenyl]-3-yl)-3,6-dihydro-2,2,6,6-tetramethyl-2H-pyran-3-one; 5-(acetyloxy)-4-(4'-chloro-4-ethyl-2'-fluoro[1,1'-biphenyl]-3-yl)-3,6-dihydro-2,2,6,6-tetramethyl-2H-pyran-3-one (CAS No.: 1312340-82-1); 5-(acetyloxy)-4-(2',4'-dichloro-4-ethyl[1,1'-biphenyl]-3-yl)-3,6-dihydro-2,2,6,6-tetramethyl-2H-pyran-3-one (CAS No.: 1033760-55-2); 4-(4'-chloro-4-cyclopropyl-2'-fluoro[1,1'-biphenyl]-3-yl)-5,6-dihydro-2,2,6,6-tetramethyl-5-oxo-2H-pyran-3-yl methyl carbonate (CAS No.: 1312337-51-1); 4-(2',4'-dichloro-4-cyclopropyl-[1,1'-biphenyl]-3-yl)-5,6-dihydro-2,2,6,6-tetramethyl-5-oxo-2H-pyran-3-yl methyl carbonate; 4-(4'-chloro-4-ethyl-2'-fluoro[1,1'-biphenyl]-3-yl)-5,6-dihydro-2,2,6,6-tetramethyl-5-oxo-2H-pyran-3-yl methyl carbonate (CAS No.: 1312340-83-2); and 4-(2',4'-dichloro-4-ethyl[1,1'-biphenyl]-3-yl)-5,6-dihydro-2,2,6,6-tetramethyl-5-oxo-2H-pyran-3-yl methyl carbonate (CAS No.: 1033760-58-5);
(6) GS (Glutamine Synthetase) inhibitors: e.g., bialaphos/bilanafos, bilanaphos-natrium, glufosinate-ammonium, glufosinate, and glufosinate-P;
(7) PDS (Phytoene Desaturase) inhibitors: e.g., flurochloridone, flurtamone, beflubutamid-M, norflurazon, fluridone, diflufenican, picolinafen, and 4-[3-(trifluoromethyl)phenoxy]-2-[4-(trifluoromethyl)phenyl]pyrimidine (CAS NO: 180608-33-7);
(8) DHPS (Dihydropteroate Synthase) inhibitors: e.g., asulam;
(9) DXPS (Deoxy-D-Xylulose Phosphate Synthase) inhibitors: e.g., bixlozone, and clomazone;
(10) HST (Homogentisate Solanesyltransferase) inhibitors: e.g., cyclopyrimorate;
(11) SPS (Solanesyl Diphosphate Synthase) inhibitors: e.g., aclonifen;
(12) Cellulose Synthesis inhibitors: e.g., indaziflam, triaziflam, chlorthiamid, dichlobenil, isoxaben, flupoxam, 1-cyclohexyl-5-pentafluorophenyloxy-1⁴-[1,2,4,6]thiatriazin-3-ylamine (CAS NO: 175899-01-1), and the azines disclosed in CN109688807A;

(13) VLCFAS (Very Long-Chain Fatty Acid Synthesis) inhibitors: including but not limited to the following types,
1) Chloroacetamides, e.g., acetochlor, alachlor, butachlor, dimethachlor, dimethenamid, dimethenamid-P, metazachlor, metolachlor, S-metolachlor, pethoxamid, pretilachlor, propachlor, propisochlor, and thenylchlor;
2) α-Oxyacetamides, e.g., flufenacet, and mefenacet;
3) α-Thioacetamides, e.g., anilofos, and piperophos;
4) Azolyl-carboxamides, e.g., cafenstrole, fentrazamide, and ipfencarbazone;
5) Benzofuranes, e.g., benfuresate, and ethofumesate;
6) Isoxazolines, e.g., fenoxasulfone, and pyroxasulfone;
7) Ethylene oxides, e.g., indanofan, and tridiphane;
8) Thiocarbamates, e.g., cycloate, dimepiperate, S-ethyl dipropyl(thiocarbamate) (EPTC), esprocarb, molinate, orbencarb, prosulfocarb, thiobencarb/benthiocarb, triallate, vernolate, isoxazolines of formulas II.1, II.2, II.3, II.4, II.5, II.6, II.7, II.8 and II.9, and other isoxazoline compounds mentioned in patents such as WO 2006/024820, WO 2006/037945, WO 2007/071900 and WO 2007/096576. 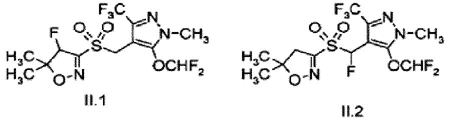 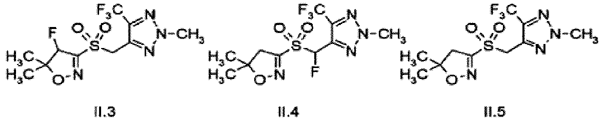 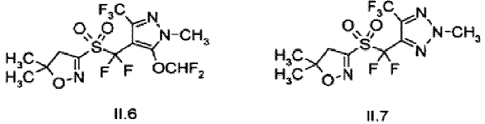 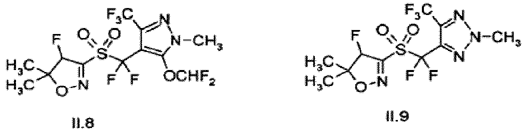
(14) Fatty acid thioesterase inhibitors: e.g., cinmethylin and methiozolin;
(15) Serine threonine protein phosphatase inhibitors: e.g., endothall;
(16) Lycopene cyclase inhibitors: e.g., amitrole.
(17) Microtubule Assembly inhibitors: e.g., benefin/benfluralin, butralin, dithiopyr, thiazopyr, ethalfluralin, prodiamine, butamifos, oryzalin, pendimethalin, chlorthal-dimethyl/DCPA, DMPA ( 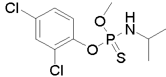 ), trifluralin, propyzamide/pronamide, and dinitramine (CAS No.: 29091-05-2);
(18) Auxin Mimics: e.g., 2,4,5-trichlorophenoxyacetic acid (2,4,5-T), 2,4-dichlorophenoxyacetic acid (2,4-D), 2,4-dichlorophenoxybutyric acid (2,4-DB), 3,4-dichlorophenoxybutyric acid (3,4-DB), 2,4-DEB (CAS No.: 94-83-7), 4-chlorophenoxyacetic acid (4-CPA), 4-CPP (CAS No.: 3307-39-9), cloprop (CAS No.: 101-10-0), clofop (CAS NO: 26129-32-8), clomeprop, dichlorprop, (4-chloro-2-methylphenoxy)acetic acid (MCPA), 2-methyl-4-chlorophenoxybutyric acid (MCPB), mecoprop, aminopyralid, clopyralid, aminocyclopyrachlor, florpyrauxifen -benzyl, halauxifen, quinclorac, benazolin-ethyl, picloram 4-amino-3,5,6-trichloropicolinic acid, chloramben, quinmerac, fluroxypyr, triclopyr and dicamba;
(19) D1 Serine 264 binders (and other non-histidine 215 binders): e.g., ametryne, atrazine, cyanazine, dimethametryn, prometon, prometryn, propazine, simazine, terbuthylazine, terbutryne, chlorotoluron, diuron, fluometuron, isoproturon, linuron, metobromuron, methabenzthiazuron (CAS No.: 18691-97-9), monuron, tebuthiuron, hexazinone, metamitron, metribuzin, bromacil, lenacil, terbacil, chloridazon/pyrazon, amicarbazone, desmedipham, phenmedipham, chloranocryl/dicryl (CAS No.: 2164-09-2), propanil, chlorazine (CAS No.: 580-48-3), cyprazine (CAS No.: 22936-86-3), trietazine (CAS No.: 1912-26-1), methoprotryne (CAS No.: 841-06-5), and simetryn;
(20) D1 Histidine 215 binders: e.g., bromoxynil, ioxynil, pyridate, and bentazone;
(21) Auxin Transport inhibitors: e.g., diflufenzopyr (CAS No.: 109293-97-2), and naptalam;
(22) PS | Electron Diversion: e.g., diquat, and paraquat;
(23) Inhibition of Microtubule Organization: e.g., carbetamide (CAS No.: 16118-49-3), barban (CAS No.: 101-27-9), and chlorpropham;
(24) Uncouplers: e.g., dinoseb, and 4,6-dinitro-o-cresol (DNOC);
(25) Others: e.g., bensulide, bromobutide, cumyluron, difenzoquat, pyributicarb (CAS No.: 88678-67-5), disodium methylarsenate (DSMA), sodium monomethylarsenate (MSMA), dymron/daimuron, etobenzanid, flamprop-m, fosamine, oxaziclomefone, pelargonic acid, diphenamid, naproanilide, napropamide, napropamide-m, benzofluor (CAS No.: 68672-17-3), cambendichlor (CAS No.: 56141-00-5), dipropalin (CAS No.: 1918-08-7), etnipromid (CAS No.: 76120-02-0), bromobonil (CAS No.: 25671-46-9), monisouron (CAS No.: 55807-46-0), bromofenoxim (CAS No.: 13181-17-4), clacyfos (CAS No.: 215655-76-8), chlorazifop (CAS No.: 60074-25-1), kuicaoxi, brompyrazon (CAS No.: 304284-0), ethiozin, methiuron (CAS No.: 21540-35-2), metoxuron (CAS No.: 19937-59-8), tetrafluron (CAS No.: 27954-37-6), thidiazuron (CAS No.: 51707-55-2), acrolein, and funaihecaoling.

In one specific embodiment, another herbicide (component B) mixed with Compound 1 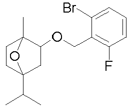 (component A) is selected from one or more of the following compounds: bensulfuron-methyl (CAS No.: 83055-99-6), pyrazosulfuron-ethyl (CAS No.: 93697-74-6), penoxsulam (CAS No.: 219714-96-2), thifensulfuron-methyl (CAS No.: 79277-27-3), flumetsulam (CAS No.: 98967-40-9), 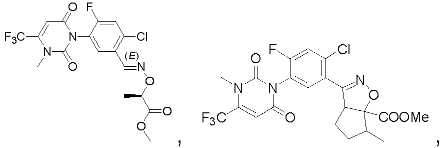 oxadiazon (CAS No.: 19666-30-9), oxadiargyl (CAS No.: 39807-15-3), oxyfluorfen (CAS No.: 42874-03-3), pyraclonil (CAS No.: 158353-15-2), flumioxazin (CAS No.: 103361-09-7), propanil (CAS No.: 709-98-8), bentazone (CAS No.: 25057-89-0), isoproturon (CAS No.: 34123-59-6), chlorotoluron (CAS No.: 15545-48-9), metribuzin (CAS No.: 21087-64-9), atrazine (CAS No.: 1912-24-9), terbuthylazine (CAS No.: 5915-41-3), prometryn (CAS No.: 7287-19-6), 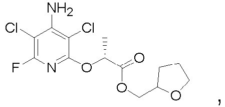 florpyrauxifen-benzyl (CAS No.: 1390661-72-9), fluroxypyr (CAS No.: 69377-81-7), MCPA-isooctyl (CAS No.: 26544-20-7), 2,4-D-isooctyl (CAS No.: 25168-26-7), 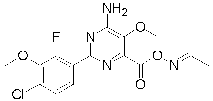 , tripyrasulfone (CAS No.: 1911613-97-2), 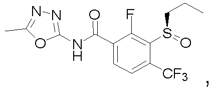 bipyrazone (CAS No.: 1622908-18-2), fenpyrazone (CAS No.: 1992017-55-6), mesotrione (CAS No.: 104206-82-8), benzobicyclon (CAS No.: 156963-66-5), tefuryltrione (CAS No.: 473278-76-1), isoxaflutole (CAS No.: 141112-29-0), acetochlor (CAS No.: 34256-82-1), pretilachlor (CAS No.: 51218-49-6), butachlor (CAS No.: 23184-66-9), mefenacet (CAS No.: 73250-68-7), fentrazamide (CAS No.: 158237-07-1), anilofos (CAS No.: 64249-01-0), pyroxasulfone (CAS No.: 447399-55-5), triallate (CAS No.: 2303-17-5), pendimethalin (CAS No.: 40487-42-1), butralin (CAS No.: 33629-47-9), diflufenican (CAS No.: 83164-33-4), beflubutamid-M (CAS No.: 113614-09-8), 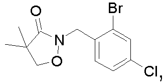 clomazone (CAS No.: 81777-89-1), bixlozone (CAS No.: 81777-95-9), oxaziclomefone (CAS No.: 153197-14-9), cyhalofop-butyl (CAS No.: 122008-85-9), metamifop (CAS No.: 256412-89-2), and profoxydim (CAS No.: 139001-49-3).

Generally, if the herbicidal compounds as described herein, which can be employed in the context of the present invention, are capable of forming geometrical isomers, for example, E/Z isomers, it is possible to use both, the pure isomers and mixtures thereof, in the compositions according to the invention. If the herbicidal compounds as described herein have one or more chiral centers (the compound 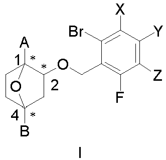 shown in Formula I has at least 3 chiral centers, C1*, C2* and C4*) and, as a consequence, are present as enantiomers or diastereomers, it is possible to use both, the pure enantiomers and diastereomers and mixtures thereof, in the compositions according to the invention. If the herbicidal compounds as described herein have ionizable functional groups, they may also be employed in the form of their agriculturally acceptable salts. In general, the salts of those cations and the acid addition salts of those acids are suitable, the cations and anions of which have no adverse effect on the activity of the active compounds, respectively. Preferred cations are the ions of alkali metals, preferably of lithium, sodium, and potassium; ions of alkaline earth metals, preferably of calcium and magnesium; and ions of transition metals, preferably of manganese, copper, zinc, and iron; further ammonium and substituted ammonium in which 1 to 4 hydrogen atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl, phenyl or benzyl, preferably ammonium, methylammonium, isopropylammonium, dimethylammonium, diisopropylammonium, trimethylammonium, heptylammonium, dodecylammonium, tetradecylammonium, tetramethylammonium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt); furthermore, phosphonium ions, sulfonium ions, preferably tri(C₁-C₄-alkyl)sulfonium such as trimethylsulfonium, and sulfoxonium ions, preferably tri(C₁-C₄-alkyl)sulfoxonium, and finally polyamines such as N,N-bis-(3-aminopropyl)methylamine and diethylenetriamine. Available anions of acid addition salts are mainly chloride, bromide, fluoride, iodide, bisulfate, methyl sulfate, sulfate, dihydrogen phosphate, hydrogen phosphate, nitrate, bicarbonate, carbonate, hexafluorosilicate, hexafluorophosphate, benzoate, and also anions of C₁-C₄-alkanoic acids, preferably formate, acetate, propionate, and butyrate.

The herbicidal compounds as described herein having a carboxyl group can be employed in the form of an acid, in the form of an agriculturally suitable salt as mentioned above or else in the form of an agriculturally acceptable derivative, for example as amides such as mono- and di-C₁-C₆-alkylamides or arylamides, as esters such as allyl esters, propargyl esters, C₁-C₁₀-alkyl esters, alkoxyalkyl esters, tefuryl ((tetrahydrofuran-2-yl)methyl) esters and also as thioesters such as C₁-C₁₀-alkylthio esters. Preferred mono- and di-C₁-C₆ alkyl amides are methyl- and dimethylamide. Preferred arylamides are, for example, N-anilide and 2-chloroanilide. Preferred alkyl esters are, for example, methyl, ethyl, propyl, isopropyl, butyl, isobutyl, pentyl, mexyl (1-methylhexyl), meptyl (1-methylheptyl), heptyl, octyl or isooctyl (2-ethylhexyl) esters. Preferred C₁-C₄ alkoxy-C₁-C₄-alkyl esters are linear or branched C₁-C₄ alkoxyethyl esters such as 2-methoxylethyl esters, 2-ethoxylethyl esters, 2-butoxyethtyl (butotyl) esters, 2-butoxypropyl esters or 3-butoxypropyl esters. An example of linear or branched C₁-C₁₀ alkyl thioesters is ethyl thioester.

For use, the commercially available formulations are, if needed, diluted in customary manners, for example using water in the cases of wettable powders, emulsifiable concentrates, dispersions and water-dispersible granules. For products in the form of dusts, granules for soil application, or solutions for broadcasting and spray, usually no further dilution with inert materials is required prior to use. The application amount of the compounds of Formula I required varies with the external conditions such as temperature, humidity, the nature of the herbicide used and the like. It can vary within wide limits, for example between 0.001 and 1.0 kg a.i./ha or more of active substances, but it is preferably between 0.005 and 750 g a.i./ha.

### Detailed Embodiments of the Invention

The following examples are used to illustrate the present invention and should not be regarded as limiting the present invention in any way. The scope of rights claimed by the present invention is described by the claims.

In view of the economy and diversity of the compounds, some compounds were preferably synthesized. Among the many synthesized compounds, selected ones are listed in Table 1 below. The specific compound structure and corresponding compound information are as set forth in Table 1. The compounds in Table 1 are only to better illustrate the present invention, but do not limit the present invention. For a person skilled in the art, it should not be understood that the scope of the above-mentioned subject matters of the present invention is limited to the following compounds.

**Table 1 Compound structure and ¹H NMR thereof**

| 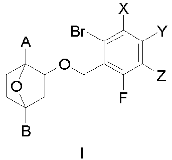 | | | | | | |
|---|---|---|---|---|---|---|
| **No.** | **A** | **B** | **X** | **Y** | **Z** | **¹H NMR** |
| 1 | CH₃ | CH(CH₃)₂ | H | H | H | ¹H NMR (500 MHz, DMSO-*d*₆) δ 7.54-7.52 (m, 1H), 7.44 - 7.34 (m, 1H), 7.32-7.28 (m, 1H), 4.56 - 4.45 (m, 2H), 3.65-3.58 (m, 1H), 2.04-1.90 (m, 2H), 1.65 - 1.37 (m, 5H), 1.30 (s, 3H), 0.91 (dd, *J* = 7.0, 2.5 Hz, 6H). |
| 2 | CH₃ | CH(CH₃)₂ | F | H | H | |
| 3 | CH₃ | CH(CH₃)₂ | Cl | H | H | ¹H NMR (300 MHz, Chloroform-d) δ 7.40-7.34 (m, 1H), 7.01-6.92 (m, 1H), 4.66-4.51 (m, 2H), 3.52 (dd, *J =* 6.9, 2.4 Hz, 1H), 2.12-2.03 (m, 1H), 2.01-1.92 (m, 1H), 1.64 - 1.42 (m, 5H), 1.40 (s, 3H), 0.97-0.91 (m, 6H). |
| 4 | CH₃ | CH(CH₃)₂ | Br | H | H | |
| 5 | CH₃ | CH(CH₃)₂ | CH₃ | H | H | ¹H NMR (300 MHz, Chloroform-d) δ 7.21-7.13 (m, 1H), 6.97-6.88 (m, 1H), 4.67 -4.57(m, 2H), 3.54 (dd, *J =* 6.9, 2.4 Hz, 1H), 2.38 (s, 3H), 2.14 - 2.06 (m, 1H), 2.05 - 1.95 (m, 1H), 1.63-1.60 (m, 1H), 1.59 - 1.44 (m, 4H), 1.43 (s, 3H), 0.97 (t, *J =* 6.9 Hz, 6H). |
| 6 | CH₃ | CH(CH₃)₂ | CH₂CH₃ | H | H | |
| 7 | CH₃ | CH(CH₃)₂ | OCH₃ | H | H | |
| 8 | CH₃ | CH(CH₃)₂ | H | F | H | |
| 9 | CH₃ | CH(CH₃)₂ | H | Cl | H | |
| 10 | CH₃ | CH(CH₃)₂ | H | Br | H | |
| 11 | CH₃ | CH(CH₃)₂ | H | CH₃ | H | |
| 12 | CH₃ | CH(CH₃)₂ | H | CH₂CH₃ | H | |
| 13 | CH₃ | CH(CH₃)₂ | H | OCH₃ | H | |
| 14 | CH₃ | CH(CH₃)₂ | H | H | F | ¹H NMR (300 MHz, Chloroform-d) δ 7.34-7.24 (m, 1H), 7.08-6.96 (m, 1H), 4.63 (dd, *J* = 10.5, 2.7 Hz, 1H), 4.52 (dd, *J* = 10.5, 2.7 Hz, 1H), 3.54 (dd, *J* = 6.6, 2.4 Hz, 1H), 2.15-2.04 (m, 1H), 2.03-1.95 (m, 1H), 1.67 - 1.43 (m, 5H), 1.42 (s, 3H), 1.02-0.90 (m, 6H). |
| 15 | CH₃ | CH(CH₃)₂ | H | H | Cl | ¹H NMR (300 MHz, Chloroform-d) δ 7.35-7.29 (m, 1H), 7.25 - 7.19 (m, 1H), 4.68 - 4.50 (m, 2H), 3.54 (dd, *J* = 6.9, 2.4 Hz, 1H), 2.19-2.06 (m, 1H), 2.04-1.96 (m, 1H), 1.68 - 1.46 (m, 5H), 1.43 (s, 3H), 1.02-0.94 (m, 6H). |
| 16 | CH₃ | CH(CH₃)₂ | H | H | Br | ¹H NMR (300 MHz, Chloroform-d) δ 7.36 (dd, *J* = 8.7, 6.9 Hz, 1H), 7.27 - 7.22 (m, 1H), 4.70 - 4.46 (m, 2H), 3.54 (dd, *J* = 6.9, 2.4 Hz, 1H), 2.17-2.06 (m, 1H), 2.05-1.97 (m, 1H), 1.66 - 1.45 (m, 5H), 1.43 (s, 3H),1.05-0.87 (m, 6H). |
| 17 | CH₃ | CH(CH₃)₂ | H | H | CH₃ | ¹H NMR (300 MHz, Chloroform-*d*) δ 7.26 - 7.21 (m, 1H), 706 - 6.94 (m, 1H), 4.71 - 4.47 (m, 2H), 3.55 (dd, *J* = 6.9, 2.4 Hz, 1H), 2.22 (d, *J* = 2.1 Hz, 3H), 2.14 - 1.95 (m, 2H), 1.69-1.61 (m, 1H), 1.59 - 1.46 (m, 4H), 1.43 (s, 3H), 0.97 (t, *J* = 6.9 Hz, 6H). |
| 18 | CH₃ | CH(CH₃)₂ | H | H | CH₂CH₃ | |
| 19 | CH₃ | CH(CH₃)₂ | H | H | OCH₃ | ¹H NMR (300 MHz, Chloroform-d) δ 7.33 - 7.22 (m, 1 H), 6.87 - 6.74 (m, 1H), 4.69 - 4.48 (m, 2H),3.87 (s, 3H), 3.53 (dd, *J* = 6.9, 2.4 Hz, 1H), 2.17 - 2.05 (m, 1H), 2.04-1.94 (m, 1 H), 1.65-1.44 (m, 5H), 1.41 (s, 3H), 1.01-0.94 (m, 6H). |
| 20 | CH₃ | CH(CH₃)₂ | CH₃ | Cl | H | |
| 21 | CH₃ | CH(CH₃)₂ | CH₃ | Br | H | |
| 22 | CH₃ | CH(CH₃)₂ | Cl | F | H | |
| 23 | CH₃ | CH(CH₃)₂ | Cl | OCH₃ | H | |
| 24 | CH₃ | CH(CH₃)₂ | F | OCH₃ | H | |
| 25 | CH₃ | CH(CH₃)₂ | OCH₃ | Br | H | |
| 26 | CH₃ | CH(CH₃)₂ | H | Cl | CH₃ | |
| 27 | CH₃ | CH(CH₃)₂ | H | CH₃ | Br | |
| 28 | CH₃ | CH(CH₃)₂ | H | Cl | OCH₃ | |
| 29 | CH₃ | CH(CH₃)₂ | H | CH₃ | Cl | |
| 30 | CH₃ | CH(CH₃)₂ | H | F | CH₃ | |
| 31 | CH₃ | CH(CH₃)₂ | H | OCH₃ | Br | |
| 32 | CH₃ | CH(CH₃)₂ | OCH₃ | H | Cl | |
| 33 | CH₃ | CH(CH₃)₂ | Cl | H | Br | |
| 34 | CH₃ | CH(CH₃)₂ | F | H | OCH₃ | |
| 35 | CH₃ | CH(CH₃)₂ | Cl | H | CH₃ | |
| 36 | CH₃ | CH(CH₃)₂ | Br | H | Cl | |
| 37 | CH₃ | CH(CH₃)₂ | CH₃ | H | Br | |
| 38 | CH₃ | CH(CH₃)₂ | Cl | Br | CH₃ | |
| 39 | CH₃ | CH(CH₃)₂ | CH₃ | F | Br | |
| 40 | CH₃ | CH(CH₃)₂ | OCH₃ | CH₃ | Cl | |
| 41 | CH₃ | CH(CH₃)₂ | F | CH₃ | Cl | |
| 42 | CH₃ | CH(CH₃)₂ | CN | H | H | |
| 43 | CH₃ | CH(CH₃)₂ | NO₂ | H | H | |
| 44 | CH₃ | CH(CH₃)₂ | 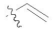 | H | H | |
| 45 | CH₃ | CH(CH₃)₂ | 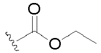 | H | H | |
| 46 | CH₃ | CH(CH₃)₂ | 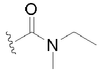 | H | H | |
| 47 | CH₃ | CH(CH₃)₂ | 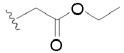 | H | H | |
| 48 | CH₃ | CH(CH₃)₂ | 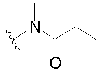 | H | H | |
| 49 | CH₃ | CH(CH₃)₂ | 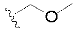 | H | H | |
| 50 | CH₃ | CH(CH₃)₂ | H | 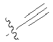 | H | |
| 51 | CH₃ | CH(CH₃)₂ | H | NO₂ | H | |
| 52 | CH₃ | CH(CH₃)₂ | H | OH | H | |
| 53 | CH₃ | CH(CH₃)₂ | H | SH | H | |
| 54 | CH₃ | CH(CH₃)₂ | H | SCH₃ | H | |
| 55 | CH₃ | CH(CH₃)₂ | H | SOCH₃ | H | |
| 56 | CH₃ | CH(CH₃)₂ | H | SO₂CH₃ | H | |
| 57 | CH₃ | CH(CH₃)₂ | H | NH₂ | H | |
| 58 | CH₃ | CH(CH₃)₂ | H | 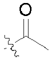 | H | |
| 59 | CH₃ | CH(CH₃)₂ | H | 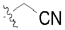 | H | |
| 60 | CH₃ | CH(CH₃)₂ | H | 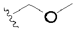 | H | |
| 61 | CH₃ | CH(CH₃)₂ | H | 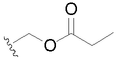 | H | |
| 62 | CH₃ | CH(CH₃)₂ | H | 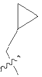 | H | |
| 63 | CH₃ | CH(CH₃)₂ | H | 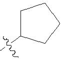 | H | |
| 64 | CH₃ | CH(CH₃)₂ | H | 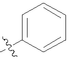 | H | |
| 65 | CH₃ | CH(CH₃)₂ | H | 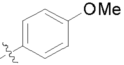 | H | |
| 66 | CH₃ | CH(CH₃)₂ | H | 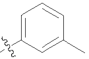 | H | |
| 67 | CH₃ | CH(CH₃)₂ | H | 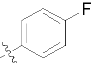 | H | |
| 68 | CH₃ | CH(CH₃)₂ | H | 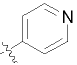 | H | |
| 69 | CH₃ | CH(CH₃)₂ | H | 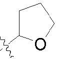 | H | |
| 70 | CH₃ | CH(CH₃)₂ | H | 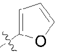 | H | |
| 71 | CH₃ | CH(CH₃)₂ | H | 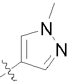 | H | |
| 72 | CH₃ | CH(CH₃)₂ | H | H | 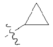 | |
| 73 | CH₃ | CH(CH₃)₂ | H | H | CN | |
| 74 | CH₃ | CH(CH₃)₂ | H | H | COCH₃ | |
| 75 | CH₃ | CH₃ | H | H | H | |
| 76 | CH₃ | CH₂CH₃ | H | H | H | |
| 77 | CH₂CH₃ | CH₃ | H | H | H | |
| 78 | CH₂CH₃ | CH₂CH₃ | H | H | H | |
| 79 | CH₂CH₃ | CH(CH₃)₂ | H | H | H | |

Tables A-H have the same structure as the above Table 1 does, except that the racemate compounds with chiral centers (the carbon atoms at C1*, C2* and C4* in the compound represented by the general formula I 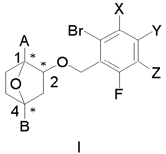 are chiral centers) are replaced by (1*R*, 2*R*, 4*R*) configuration, (1*S*, 2*R,* 4*R*) configuration, (1*R,* 2*S*, 4*R*) configuration, (1*R, 2R, 4*S) configuration, (1*S*, 2*S*, 4*R*) configuration, (1*R,* 2*S*, 4*S*) configuration, (1*S*, *2R,* 4S) configuration, and (1*S*, 2S, 4S) configuration, respectively. Moreover, in Table A, the items below the column heading "No." are sequentially described as "1(1*R, 2R,* 4*R*)-79(1*R*, 2*R,* 4*R*)", and the other tables have the same structure. For example, "1(1*R,* 2*R,* 4*R*)" corresponds to Compound "1" in Table 1 where the C1* is in *R-*configuration, C2* is in *R*-configuration and C4* is in *R*-configuration.

Several methods for preparing the compounds of the present invention are illustrated in detail in the following schemes and examples. Raw materials can be purchased on the market or can be prepared by methods known in literatures or as shown in the detailed description. A person skilled in the art should understand that other synthetic routes may also be used to synthesize the compounds of the present invention. Although the specific raw materials and conditions in the synthetic route have been illustrated below, they may be easily replaced with other similar raw materials and conditions. Various isomers and the like of the compounds resulted from these modifications or variants of the preparation method of the present invention are all included in the scope of the present invention. In addition, the preparation methods as described below can be further modified according to the disclosure of the present invention using conventional chemical methods well known to a person skilled in the art. For example, protection for appropriate groups during the reaction, etc.

Examples of methods provided below are used to enhance further understanding of the preparation methods of the present invention, and the specific substances, varieties and conditions employed are determined to be further illustrations of the present invention and not to limit its reasonable scope. Reagents used in the synthesis of the compounds shown in the following tables are either commercially available or can be easily prepared by a person of ordinary skill in the art.

Examples of representative compounds are as follows. The synthesis methods of other compounds are similar and will not be described in detail here.

### 1. Synthesis of Compound 1

Compound 1-2 (0.37 g, 2.2 mmol, 1.1 eq) and NaH (96 mg, 2.4 mmol, 1.2 eq) were dissolved in 20 ml of DMF, added with Compound 1-1 (0.53 g, 2.0 mmol, 1 eq) after stirring for 0.5 h at room temperature, heated up to 40°C and reacted for 5 h. The reaction was monitored until the raw materials had been reacted completely. The reaction solution was poured into 20 ml of ice water, and repeatedly extracted with 20 ml of ethyl acetate for three times. The organic phase was concentrated by decompression, isolated and purified through silica gel column to obtain Compound 1 (0.4 g, 57% yield).

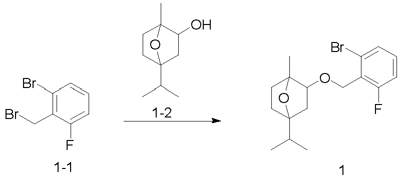

### 2. Synthesis of Compound 3

Compound 3-1 (1.13 g, 4.4 mmol, 1 eq) was dissolved in 20 ml of DMF, added with sodium hydride (0.21 g, 8.8 mmol, 2 eq) in ice bath, and stirred for 0.5 h at room temperature. The cinmethylin intermediate 1-2 (0.8 g, 4.4 mmol, 1.1 eq) was added and stirred for 12 h at room temperature. The reaction was monitored until the starting materials had been reacted completely. The reaction solution was added with water, extracted three times with ethyl acetate, washed three times with saturated saline solution, and dried by anhydrous sodium sulfate. The sample passed through the column after stirring, and the fraction was spin-dried to obtain Compound 3 (0.92 g, 54.1% yield).

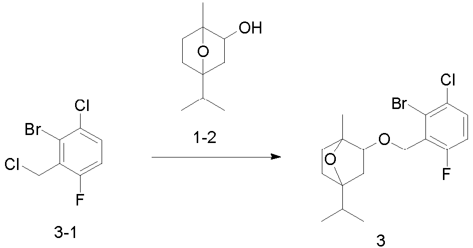

### 3. Synthesis of Compound 1(1S, 2R, 4R)

Compound 1-3 (1 eq) was added into 5 ml of DMF, added with sodium hydride (2 eq) in ice bath, and stirred for 0.5 h while remaining in ice bath. Then the reaction solution was added with Compound 1-4 (1 eq) and reacted overnight at room temperature. When the In-Process Control was completed, water was added to the reaction solution to quench the reaction and EA was added to extract twice. The organic phase was washed twice with saturated saline solution and dried by anhydrous sodium sulfate. The sample was stirred and purified by normal phase chromatography to obtain Compound 1(1*S*, 2*R*, 4*R*).

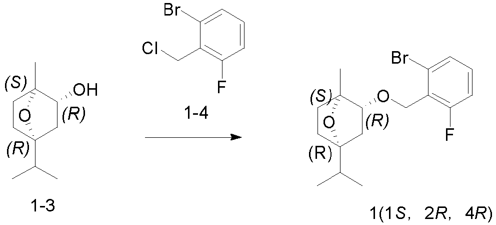

### 4. Synthesis of Compound 1(1S, 2S, 4R)

Compound 1-5 (1 eq) was added into 5 ml of DMF, added with sodium hydride (2 eq) in ice bath, and stirred for 0.5 h while remaining in ice bath. The reaction solution was added with Compound 1-4 (1 eq) and reacted overnight at room temperature. When the In-Process Control was completed, water was added to the reaction solution to quench the reaction and EA was added to extract twice. The organic phase was washed twice with saturated saline solution and dried with anhydrous sodium sulfuric acid. The sample was stirred and purified by normal phase chromatography to obtain Compound 1(1*S*, 2*S*, 4*R*).

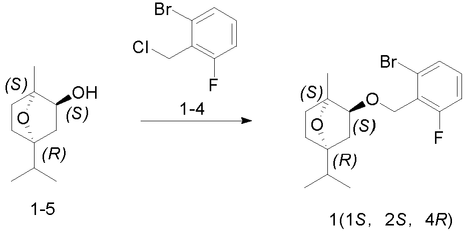

Evaluation of Biological Activity:
(1) Pre-emergence test experiment:
   The activity level criteria for plant damage (i.e., growth control rate) are as follows:
   Level 9: completely dead;
   Level 8: growth control rate is greater than or equal to 90% and less than 100%;
   Level 7: growth control rate is greater than or equal to 80% and less than 90%;
   Level 6: growth control rate is greater than or equal to 70% and less than 80%;
   Level 5: growth control rate is greater than or equal to 50% and less than 70%;
   Level 4: growth control rate is greater than or equal to 30% and less than 50%;
   Level 3: growth control rate is greater than or equal to 20% and less than 30%;
   Level 2: growth control rate is greater than or equal to 10% and less than 20%;
   Level 1: growth control rate is less than 10%;
   Level 0: no effect

The above growth control rates are fresh weight control rates.
Monocotyledonous and dicotyledonous weed seeds as well as major crop seeds (wheat, corn, rice, soybean, peanut, cotton, oilseed rape, millet, and sorghum) were placed in plastic pots filled with soil, and then covered with 0.5-2 cm of soil. The test compounds of the present invention were individually dissolved with acetone, then added with Tween 80, diluted with a certain amount of water to obtain a solution of a certain concentration, and sprayed immediately after sowing. After drug application, cultivation was conducted for 4 weeks in a greenhouse and then the experimental results were observed. The doses of the applied compounds were 3.75, 7.5, 15, 30, 60, and 120 g a.i./ha. Representative data were listed in Table 2.

**Table 2 Results of pre-emergence test experiment**

| **Compound No.** | ***Lolium perenne*** | ***Alopecurus aequalis*** | ***Beckmannia syzigachne*** | ***Setaria viridis*** | **Wheat** | **Peanut** | **Soybean** | **Corn** | **Cotton** |
|---|---|---|---|---|---|---|---|---|---|
| | **30 g a.i./ha** | **30 g a.i./ha** | **60 g a.i./ha** | **30 g a.i./ha** | **60 g a.i./ha** | **60 g a.i./ha** | **60 g a.i./ha** | **60 g a.i./ha** | **60 g a.i./ha** |
| 1 | 7 | 7 | 9 | 9 | 2 | 0 | 0 | 0 | 0 |
| Control compound A | 1 | 5 | 8 | 3 | 2 | N | N | 1 | N |
| Control compound B | 6 | 5 | 5 | 3 | 3 | N | N | 2 | N |
| Control compound C | 7 | 7 | 8 | 5 | 8 | N | N | 4 | N |

Note: N representing no data; Control compound A: 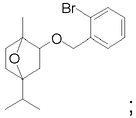 Control compound B: 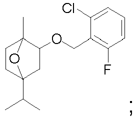 Control compound C:

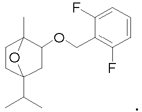

From the above, it can be found that the compound of the present invention has excellent effects, significantly higher herbicidal activity than that of Control compounds A and B, and improved crop safety compared with Control Compound C while maintaining high herbicidal activity, that is, having good selectivity, especially when using Control compound C for sealing weeding, unacceptable phytotoxicity is posed to wheat.

Meanwhile, it has been found after tests on major weeds in wheat and rice fields that the compounds of the present invention generally have good weed control effects, particularly extremely high activity against broad-leaf weeds and *Cyperaceae* with resistance to ALS inhibitors being noticed, including *Monochoria vaginalis, Cyperus difformis, Ludwigia prostrata, Lolium perenne, Alopecurus aequalis, Beckmannia syzigachne,* etc., which have excellent commercial values.

### (2) Safety evaluation on transplanted rice and direct sowing rice and weed control effect evaluation in rice fields:

After rice field soil was loaded into a 1/1,000,000 ha pot, the seeds of *Echinochloa crusgalli, Echinochloa phyllopogon, Monochoria vaginalis, Leptochloa chinensis,* and *Ludwigia prostrata* were sowed and gently covered with soil, then left to stand in a greenhouse with water storage of 0.5-1 cm deep. The tuber of *Sagittaria trifolia* was planted in the next day or 2 days later. The water storage was kept at a depth of 3-4 cm thereafter. When the *Echinochloa crusgalli, Echinochloa phyllopogon,* and *Leptochloa chinensis* reached their 0.5-leaf stages and the *Sagittaria trifolia* reached the time point of the primary leaf stage, WP or SC aqueous dilutions of the compounds of the present invention formulated according to common formulation methods were homogeneously dripped to the specified effective amount using pipettes for treatment.

To simulate a field of direct sowing rice, a 5-cm water layer was added to the container first, fully mixed with mud to form slurry, and then rice seeds were scattered in. After the slurry became clear, the water layer was removed. The soil was kept moist and applied with drug solution evenly by spraying. The soil was rehydrated to 3-cm deep 3-4 days after drug application.

In addition, after being loaded into the 1/1,000,000 ha pot, the rice field soil was leveled to keep the water storage at a depth of 2-3 cm. The rice (japonica rice) at 3-leaf stage was transplanted at a transplanting depth of 2-3 cm the next day. The compounds of the present invention were treated in the same manner as above on the 3^{rd} day after the transplantation.

The doses of the compounds used in the above-mentioned experiments were 3.75, 7.5, 15, 30, 60, and 120 g a.i./ha. The development conditions of the weeds on the 14^{th} day after the drug treatment and the development conditions of rice on the 7^{th} day after the drug treatment were observed with the naked eye, respectively. The effects were evaluated according to the above-mentioned activity level criteria. Representative data were listed in Table 3.

**Table 3 Experimental results of safety evaluation on transplanted rice and direct sowing rice and weed control effect evaluation in rice fields**

| **Compound No.** | ***Echinochloa phyllopogon*** | ***Echinochloa crusgalli*** | **Rice** | **Rice** | **Direct sowing rice** |
|---|---|---|---|---|---|
| | **7.5 g a.i./ha** | **7.5 g a.i./ha** | **30 g a.i./ha** | **60 g a.i./ha** | **60 g a.i./ha** |
| 1 | 9 | 9 | 0 | 1 | 1 |
| 1(1*R,* 2*S,* 4*S*) | N | 9 | 0 | 1 | 1 |
| 1(1*S*, 2*R,* 4*R*) | N | 9 | 0 | 1 | 1 |
| Control compound A | 5 | 6 | 2 | 3 | 4 |
| Control compound B | 7 | 8 | 3 | 3 | 4 |
| Control compound C | N | N | 7 | 9 | 9 |

| | | | | | |
|---|---|---|---|---|---|
| Note: *Echinochloa crusgalli, Echinochloa phyllopogon* and *Monochoria vaginalis* were collected from Heilongjiang, China and tested to be resistant to conventional doses of pyrazosulfuron. | | | | | |

Thus, compared with Control compounds A, B and C, the compound of the present invention have better herbicidal activity and/or rice safety.

### (3) Activity test of compositions:

The required active ingredient B was purchased from a reagent company or synthesized by a known method. The technical materials were all dissolved with acetone solvents and diluted with an aqueous solution of 0.1% Tween 80 emulsifier, which were to be used immediately after dilution.

### (A) Early post-emergence treatment by foliage spray (F-EP):

Weeds were cultivated by a pot culture method. A 180×140 mm plastic nutritional bowl, containing air-dried and sieved topsoil (taking up 4/5 of the bowl) collected from the field, was placed in an enamel pan, wherein the soil had an initial moisture content of 20%. Weed seeds with plump and uniform grains were selected, soaked in lukewarm water at 25°C for 6 hours, and accelerated to germinate in a 28°C biochemical incubator (in darkness). The weed seeds that had just germinated were evenly placed on the surface of the soil and then covered with 0.5-1 cm soil according to the sizes of seeds.

The culture was carried out in a controllable sunlight greenhouse at 20 to 30°C, in natural light, and with relative humidity of 57% to 72%. The soil was loam with an organic matter content of 1.63%, a pH value of 7.1, alkali-hydrolyzable nitrogen of 84.3 mg/kg, rapidly available phosphorus of 38.5 mg/kg, and rapidly available potassium of 82.1 mg/kg.

Each treatment was repeated 4 times. Four pots with 20 weed seeds sowed per pot were treated each time.

The agents were used once in total in the experiment. In the stage of 1-2 leaves, the weeds were thinned out to maintain 10 strains per treatment for later use.

The well-cultured test materials were evenly placed on a platform with an area of 0.5 m², and foliage spraying was carried out by the 3WP-2000-type walking spray tower at a dosage of 450 kg/ha and a spray pressure of 0.3 MPa. After all the solution was sprayed, the air valve was closed, and 30 seconds later, the door of the spray tower was opened, and the nutritional bowl was taken out. Then the air valve was opened, and the spray tube was cleaned by spraying 50 ml of water. The test materials were transferred to and routinely cultured in a greenhouse after the treatment.

### (B) Soil sealing treatment (S):

Weeds were cultivated in a controllable sunlight greenhouse at 20 to 30°C, in natural light, and with relative humidity of 57% to 72%. The soil was loam with an organic matter content of 1.63%, a pH value of 7.1, alkali-hydrolyzable nitrogen of 84.3 mg/kg, rapidly available phosphorus of 38.5 mg/kg, and rapidly available potassium of 82.1 mg/kg. The test soil was quantitatively filled to 3/4 of the pot, and then watered from the bottom of the pot to completely wet the soil to saturation. The test weed seeds were accelerated to just germinate, and then evenly and quantitatively sowed on the soil surface, covered with 0.5-1 cm soil according to the sizes of the seeds, and ready for use 72 hours after sowing.

Each treatment was repeated 4 times. Four pots with 20 weed seeds sowed per pot were treated each time.

The well-sowed test materials were evenly placed on a platform with an area of 0.5 m², and foliage spraying was carried out by the 3WP-2000-type walking spray tower at a dosage of 450 kg/ha and a spray pressure of 0.3 MPa. After all the solution was sprayed, the air valve was closed, and 30 seconds later, the door of the spray tower was opened, and the nutritional bowl was taken out. Then the air valve was opened, and the spray tube was cleaned by spraying 50 ml of water.

### (C) Data investigation and statistical analysis

A method for investigating absolute numbers was employed. Whole seedlings of survival weeds were cut off with a blade along the soil surface, and the fresh weight of the weeds was weighed using an analytical balance. For dead weeds, the fresh weight thereof was zero.

The investigation was performed 21 days after the treatment for once in total.

The theoretical fresh weight inhibition rate of a mixed combination of each treatment was calculated by the Gowing method (E0 = X + Y - X * Y / 100). Then compared with the measured inhibition rate (E), the type of the combined effect of the combination of the two on weeds was evaluated, which was a synergistic effect when E - E0 > 10%; an antagonistic effect when E - E0 < -10%; an additional effect when -10% ≤ E - E0 ≤ 10%. An optimal ratio was determined according to the actual control effects, characteristics of herbicides, and balance of the formulation. In the formula, X represents the fresh weight inhibition rate of the active ingredient A in a dosage of P; Y represents the fresh weight inhibition rate of the active ingredient B in a dosage of Q. The statistical results are shown in Table 4.

**Table 4 Evaluation of actual control effect and combined effect of Compound 1 mixtures on weeds**

| **A+B** | **Target of Action** | **Treatment Type** | **Dosage of A + B (g a.i./ha)** | **A:B** | **Control effect of A applied alone in a corresponding dose (%)** | **Control effect of B applied alone in a corresponding dose (%)** | **Control effect of A + B (%), E** | **E0 (%)** | **E - E0 (%)** |
|---|---|---|---|---|---|---|---|---|---|
| 1 + bensulfuron-methyl | *Cyperus glomeratus* | S | 30 + 7.5 | 4:1 | 56.1 | 27.5 | 80.2 | 68.2 | 12.0 |
| 1 + pyrazosulfuron-ethyl | *Cyperus glomeratus* | S | 30 + 7.5 | 4:1 | 56.1 | 29.8 | 79.7 | 69.2 | 10.5 |
| 1 + penoxsulam | *Cyperus glomeratus* | S | 30 + 7.5 | 4:1 | 56.1 | 26.4 | 78.5 | 67.7 | 10.8 |
| 1 + thifensulfuron-methyl | *Oxybasis glauca* | S | 60 + 7.5 | 8:1 | 19.8 | 68.6 | 87.9 | 74.8 | 13.1 |
| 1 + flumetsulam | *Oxybasis glauca* | S | 60 + 15 | 4:1 | 19.8 | 57.9 | 79.1 | 66.2 | 12.9 |
| 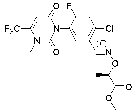 1 + | *Cyperus glomeratus* | S | 30 + 7.5 | 4:1 | 56.1 | 50.1 | 89.2 | 78.1 | 11.1 |
| 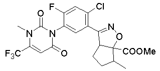 1 + | *Cyperus glomeratus* | S | 30 + 7.5 | 4:1 | 56.1 | 48.3 | 87.8 | 77.3 | 10.5 |
| 1 + oxadiazon | *Cyperus glomeratus* | S | 30 + 90 | 1:3 | 56.1 | 32.4 | 81.2 | 70.3 | 10.9 |
| 1 + oxadiargyl | *Cyperus glomeratus* | S | 30 + 45 | 2:3 | 56.1 | 42.5 | 87.6 | 74.8 | 12.8 |
| 1 + oxyfluorfen | *Cyperus glomeratus* | S | 30 + 45 | 2:3 | 56.1 | 37.2 | 84.2 | 72.4 | 11.8 |
| 1 + pyraclonil | *Cyperus glomeratus* | S | 30 + 45 | 2:3 | 56.1 | 48.6 | 89.6 | 77.4 | 12.2 |
| 1 + flumioxazin | *Oxybasis glauca* | S | 60 + 30 | 2:1 | 19.8 | 55.6 | 78.3 | 64.4 | 13.9 |
| 1 + propanil | *Cyperus glomeratus* | F-EP | 30 + 900 | 130 | 51.5 | 19.7 | 73.5 | 61.1 | 12.4 |
| 1 + bentazone | *Cyperus glomeratus* | F-EP | 30 + 300 | 1:10 | 51.5 | 43.8 | 85.6 | 72.7 | 12.9 |
| 1 + isoproturon | *Echinochloa crus-galli* var. *austrojaponensis* | F-EP | 60 + 300 | 1:5 | 52.7 | 31.3 | 82.2 | 67.5 | 14.7 |
| 1 + chlorotoluron | *Echinochloa crus-galli* var. *austrojaponensis* | F-EP | 60 + 450 | 2:15 | 52.7 | 37.6 | 85.9 | 70.5 | 15.4 |
| 1 + metribuzin | *Echinochloa crus-galli* var. *austrojaponensis* | F-EP | 60 + 150 | 2:5 | 52.7 | 42.9 | 83.3 | 73.0 | 10.3 |
| 1 + atrazine | *Echinochloa crus-galli* var. *austrojaponensis* | F-EP | 60 + 450 | 2:15 | 52.7 | 43.4 | 85.5 | 73.2 | 12.3 |
| 1 + terbuthylazine | *Echinochloa crus-galli* var. *austrojaponensis* | F-EP | 60 + 225 | 4:15 | 52.7 | 48.4 | 87.7 | 75.6 | 12.1 |
| 1 + prometryn | *Echinochloa crus-galli* var. *austrojaponensis* | F-EP | 60 + 300 | 1:5 | 52.7 | 41.0 | 89.3 | 72.1 | 17.2 |
| 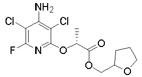 1 + | *Soliva anthemifolia* | F-EP | 60 + 150 | 2:5 | 22.8 | 64.8 | 86.4 | 72.8 | 13.6 |
| 1 + florpyrauxifen-benzyl | *Soliva anthemifolia* | F-EP | 60 + 7.5 | 8:1 | 22.8 | 60.2 | 88.3 | 69.3 | 19.0 |
| 1 + fluroxypyr | *Soliva anthemifolia* | F-EP | 60 + 75 | 4:5 | 22.8 | 66.7 | 87.8 | 74.3 | 13.5 |
| 1 + MCPA-isooctyl | *Soliva anthemifolia* | F-EP | 60 + 300 | 1:5 | 22.8 | 65.0 | 89.3 | 73.0 | 16.3 |
| 1 + 2,4-D-isooctyl | *Soliva anthemifolia* | F-EP | 60 + 225 | 4:15 | 22.8 | 61.4 | 88.7 | 70.2 | 18.5 |
| 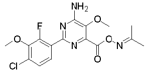 1 + | *Soliva anthemifolia* | F-EP | 60 + 6 | 10:1 | 22.8 | 69.5 | 93.0 | 76.5 | 16.5 |
| 1 + tripyrasulfone | *Echinochloa crus-galli* var. *austrojaponensis* | F-EP | 30 + 60 | 1:2 | 38.4 | 50.4 | 83.6 | 69.4 | 14.2 |
| 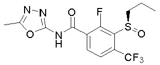 1 + | *Echinochloa crus-galli* var. *austrojaponensis* | F-EP | 30 + 30 | 1:1 | 38.4 | 56.8 | 86.4 | 73.4 | 13.0 |
| 1 + bipyrazone | *Echinochloa crus-galli* var. *austrojaponensis* | F-EP | 60 + 30 | 2:1 | 52.7 | 52.7 | 89.8 | 77.6 | 12.2 |
| 1 + fenpyrazone | *Echinochloa crus-galli* var. *austrojaponensis* | F-EP | 60 + 30 | 2:1 | 52.7 | 53.1 | 90.2 | 77.8 | 12.4 |
| 1 + mesotrione | *Echinochloa crus-galli* var. *austrojaponensis* | F-EP | 60 + 60 | 1:1 | 52.7 | 51.7 | 90.6 | 77.2 | 13.4 |
| 1 + benzobicyclon | *Echinochloa crus-galli* var. *austrojaponensis* | F-EP | 30 + 60 | 1:2 | 38.4 | 48.7 | 81.5 | 68.4 | 13.1 |
| 1 + tefuryltrione | *Echinochloa crus-galli* var. *austrojaponensis* | F-EP | 30 + 15 | 2:1 | 38.4 | 51.7 | 84.4 | 70.2 | 14.2 |
| 1 + isoxaflutole | *Echinochloa crus-galli* var. *austrojaponensis* | F-EP | 60 + 30 | 2:1 | 52.7 | 47.8 | 87.1 | 75.3 | 11.8 |
| 1 + acetochlor | *Digitaria ischaemum* | S | 60 + 300 | 1:5 | 56.4 | 41.3 | 86.7 | 74.4 | 12.3 |
| 1 + pretilachlor | *Echinochloa glabrescens* | S | 30 + 150 | 1:5 | 58.3 | 43.6 | 89.3 | 76.5 | 12.8 |
| 1 + butachlor | *Echinochloa glabrescens* | S | 30 + 300 | 1:10 | 58.3 | 41.3 | 86.1 | 75.5 | 10.6 |
| 1 + mefenacet | *Echinochloa glabrescens* | S | 30 + 150 | 1:5 | 58.3 | 37.9 | 86.1 | 74.1 | 12.0 |
| 1 + fentrazamide | *Echinochloa glabrescens* | S | 30 + 75 | 2:5 | 58.3 | 40.3 | 86.4 | 75.1 | 11.3 |
| 1 + anilofos | *Echinochloa glabrescens* | S | 30 + 75 | 2:5 | 58.3 | 37.2 | 85.7 | 73.8 | 11.9 |
| 1 + pyroxasulfone | *Digitaria ischaemum* | S | 60 + 45 | 4:3 | 56.4 | 46.3 | 87.3 | 76.6 | 10.7 |
| 1 + triallate | *Digitaria ischaemum* | S | 60 + 300 | 1:5 | 56.4 | 38.4 | 83.8 | 73.1 | 10.7 |
| 1 + pendimethalin | *Digitaria ischaemum* | S | 60 + 375 | 425 | 56.4 | 65.7 | 97.2 | 85.0 | 12.2 |
| 1 + butralin | *Digitaria ischaemum* | S | 60 + 900 | 1:15 | 56.4 | 60.2 | 95.6 | 82.6 | 13.0 |
| 1 + diflufenican | *Arivela viscosa* | S | 60 + 52.5 | 8:7 | 18.5 | 65.8 | 85.1 | 721 | 13.0 |
| 1 + beflubutamid-M | *Arivela viscosa* | S | 60 + 90 | 2:3 | 18.5 | 55.2 | 78.7 | 63.5 | 15.2 |
| 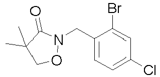 1 + | *Arivela viscosa* | S | 60 + 120 | 1:2 | 18.5 | 71.8 | 90.8 | 77.0 | 13.8 |
| 1 + clomazone | *Arivela viscosa* | S | 60 + 75 | 4:5 | 18.5 | 70.6 | 89.1 | 76.0 | 13.1 |
| 1 + bixlozone | *Arivela viscosa* | S | 60 + 120 | 1:2 | 18.5 | 74.5 | 90.2 | 79.2 | 11.0 |
| 1 + oxaziclomefone | *Cyperus glomeratus* | S | 30 + 22.5 | 4:3 | 56.1 | 58.5 | 94.2 | 81.8 | 12.4 |
| 1 + cyhalofop-butyl | *Echinochloa glabrescens* | F-EP | 30 + 150 | 1:5 | 48.7 | 46.6 | 88.7 | 72.6 | 16.1 |
| 1 + metamifop | *Echinochloa glabrescens* | F-EP | 30 + 75 | 2:5 | 48.7 | 58.8 | 94.4 | 78.9 | 15.5 |
| 1 + profoxydim | *Echinochloa glabrescens* | F-EP | 30 + 45 | 2:3 | 48.7 | 54.7 | 91.1 | 76.8 | 14.3 |

Meanwhile, it has been found after many tests that many of the compounds of the present invention and compositions thereof have good selectivity to many gramineae grasses such as zoysia grass, bermuda grass, tall fescue, bluegrass, ryegrass, seashore paspalum, etc., which are able to control many key grass weeds and broad-leaf weeds, and also show excellent selectivity and commercial values in the tests on sugarcane, oil sunflower, potato, fruit trees, vegetables and the like in different herbicide application methods.

## Claims

1. An oxabicycloalkane compound, as shown in the general formula I: 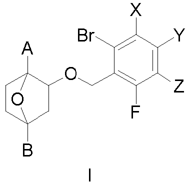 wherein, A and B each independently represent alkyl;
X, Y, and Z each independently represent hydrogen, nitro, halogen, cyano, formyl, thiocyanato, sulfhydryl, OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -alkyl-C(S)R¹, -alkyl-C(S)OR¹, -alkyl-C(O)SR¹, -alkyl-C(S)SR¹, -alkyl-SC(O)R¹, -alkyl-OC(S)R¹, -alkyl-SC(S)R¹, -O-alkyl-NR⁴R⁵, -S-alkyl-NR⁴R⁵, -alkyl-O-alkyl-NR⁴R⁵, -alkyl-S-alkyl-NR⁴R⁵, -alkyl-(C=S)ₙ-NR⁴R⁵, -NH-alkyl-NR⁴R⁵, -alkyl-OR¹, -alkyl-COR¹, -alkyl-CO₂R¹, -alkyl-OCOR¹, -alkyl-NR³COR¹, -alkyl-SO₂OR¹, -alkyl-NR³SO₂R², -alkyl-OSO₂R², -alkyl-S(O)ₘR², -alkyl-CONR⁴R⁵, -alkyl-SO₂NR⁴R⁵, NR⁴R⁵, 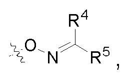 P(O)(OR⁶)₂, CH₂P(O)(OR⁶)₂, -alkyl-CN, alkyl, alkenyl, alkynyl, cycloalkyl, cycloalkylalkyl, cycloalkenyl, cycloalkenylalkyl, heterocyclyl, heterocyclylalkyl, aryl, or arylalkyl; wherein, the "alkyl", "alkenyl" or "alkynyl" are each independently unsubstituted or substituted by halogen; the "cycloalkyl", "cycloalkylalkyl", "cycloalkenyl", "cycloalkenylalkyl", "heterocyclyl", "heterocyclylalkyl", "aryl" or "arylalkyl" are each independently unsubstituted or substituted by at least one group selected from oxo, halogen, cyano, nitro, alkyl, alkenyl, alkynyl, cycloalkyl, haloalkyl, haloalkenyl, haloalkynyl, halocycloalkyl, cycloalkyl substituted by alkyl, -OR¹⁰, -SR¹⁰, -(CO)OR¹⁰, -(SO₂)R¹⁰, -N(R¹⁰)₂, or -O-alkyl-(CO)OR¹⁰, or two adjacent carbon atoms on the ring form a fused ring with -OCH₂CH₂- or -OCH₂O- that is unsubstituted or substituted by halogen;
R¹, R³, R⁴, and R⁵ each independently represent hydrogen, aryl, arylalkyl, heterocyclyl, heterocyclylalkyl, alkyl, haloalkyl, alkenyl, haloalkenyl, alkynyl, haloalkynyl, cycloalkyl, halocycloalkyl, alkoxyalkyl, cycloalkylalkyl; wherein the latter 10 groups are substituted by s substituents selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and alkoxyalkoxycarbonyl;
R² is aryl, arylalkyl, heterocyclyl, heterocyclylalkyl, alkyl, alkenyl, alkynyl, cycloalkyl, cycloalkylalkyl; wherein the latter 5 groups are substituted by s groups selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and alkoxyalkoxycarbonyl;
R⁶ is methyl or ethyl;
R⁷ and R⁸ each independently represent hydrogen, alkyl, alkenyl, or alkynyl; R⁹ is alkyl, alkenyl, or alkynyl;
R¹⁰ independently represents hydrogen, alkyl, haloalkyl, phenyl, or phenyl substituted by at least one group selected from halogen, cyano, nitro, alkyl, haloalkyl, alkoxycarbonyl, alkylthio, alkylsulfonyl, alkoxy, or haloalkoxy;
m represents 0, 1, or 2; n represents 0 or 1; s represents 0, 1, 2, 3, 4, or 5.

2. The oxabicycloalkane compound according to claim 1, wherein it is **characterized in that**,
A and B each independently represent C1-C8 alkyl;
X, Y, and Z each independently represent hydrogen, nitro, halogen, cyano, formyl, thiocyanato, sulfhydryl, OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C8 alkyl)-C(S)R¹, -(C1-C8 alkyl)-C(S)OR¹, -(C1-C8 alkyl)-C(O)SR¹, -(C1-C8 alkyl)-C(S)SR¹, -(C1-C8 alkyl)-SC(O)R¹, -(C1-C8 alkyl)-OC(S)R¹, -(C1-C8 alkyl)-SC(S)R¹, -O-(C1-C8 alkyl)-NR⁴R⁵, -S-(C1-C8 alkyl)-NR⁴R⁵, -(C1-C8 alkyl)-O-(C1-C8 alkyl)-NR⁴R⁵, -(C1-C8 alkyl)-S-(C1-C8 alkyl)-NR⁴R⁵, -(C1-C8 alkyl)-(C=S)ₙ-NR⁴R⁵, -NH-(C1-C8 alkyl)-NR⁴R⁵, -(C1-C8 alkyl)-OR¹, -(C1-C8 alkyl)-COR¹, -(C1-C8 alkyl)-CO₂R¹, -(C1-C8 alkyl)-OCOR¹, -(C1-C8 alkyl)-NR³COR¹, -(C1-C8 alkyl)-SO₂OR¹, -(C1-C8 alkyl)-NR³SO₂R², -(C1-C8 alkyl)-OSO₂R², -(C1-C8 alkyl)-S(O)ₘR², -(C1-C8 alkyl)-CONR⁴R⁵, -(C1-C8 alkyl)-SO₂NR⁴R⁵, NR⁴R⁵, 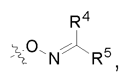 P(O)(OR⁶)₂, CH₂P(O)(OR⁶)₂, -(C1-C8 alkyl)-CN, C1-C8 alkyl, C2-C8 alkenyl, C2-C8 alkynyl, C3-C8 cycloalkyl, C3-C8 cycloalkyl C1-C8 alkyl, C3-C8 cycloalkenyl, C3-C8 cycloalkenyl C1-C8 alkyl, heterocyclyl, heterocyclyl C1-C8 alkyl, aryl, or aryl C1-C8 alkyl; wherein, the "C1-C8 alkyl", "C2-C8 alkenyl" or "C2-C8 alkynyl" are each independently unsubstituted or substituted by halogen; the "C3-C8 cycloalkyl", "C3-C8 cycloalkyl C1-C8 alkyl", "C3-C8 cycloalkenyl", "C3-C8 cycloalkenyl C1-C8 alkyl", "heterocyclyl", "heterocyclyl C1-C8 alkyl", "aryl" or "aryl C1-C8 alkyl" are each independently unsubstituted or substituted by at least one group selected form oxo, halogen, cyano, nitro, C1-C8 alkyl, C2-C8 alkenyl, C2-C8 alkynyl, C3-C8 cycloalkyl, halo C1-C8 alkyl, halo C2-C8 alkenyl, halo C2-C8 alkynyl, halo C3-C8 cycloalkyl, C3-C8 cycloalkyl substituted by C1-C8 alkyl, -OR¹⁰, -SR¹⁰, -(CO)OR¹⁰, -(SO₂)R¹⁰, -N(R¹⁰)₂, or -O-(C1-C8 alkyl)-(CO)OR¹⁰, or two adjacent carbon atoms on the ring form a fused ring with -OCH₂CH₂- or -OCH₂O- that is unsubstituted or substituted by halogen;
R¹, R³, R⁴, and R⁵ each independently represent hydrogen, aryl, aryl C1-C8 alkyl, heterocyclyl, heterocyclyl C1-C8 alkyl, C1-C8 alkyl, halo C1-C8 alkyl, C2-C8 alkenyl, halo C2-C8 alkenyl, C2-C8 alkynyl, halo C2-C8 alkynyl, C3-C8 cycloalkyl, halo C3-C8 cycloalkyl, C1-C8 alkoxy C1-C8 alkyl, C3-C8 cycloalkyl C1-C8 alkyl; wherein the latter 10 groups are substituted by s substituents selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C8 alkoxy C1-C8 alkoxycarbonyl;
R² is aryl, aryl C1-C8 alkyl, heterocyclyl, heterocyclyl C1-C8 alkyl, C1-C8 alkyl, C2-C8 alkenyl, C2-C8 alkynyl, C3-C8 cycloalkyl, C3-C8 cycloalkyl C1-C8 alkyl; wherein the latter 5 groups are substituted by s groups selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸,
and C1-C8 alkoxy C1-C8 alkoxycarbonyl;
R⁶ is methyl or ethyl;
R⁷ and R⁸ each independently represent hydrogen, C1-C8 alkyl, C2-C8 alkenyl, or C2-C8 alkynyl;
R⁹ is C1-C8 alkyl, C2-C8 alkenyl, or C2-C8 alkynyl;
R¹⁰ independently represents hydrogen, C1-C8 alkyl, halo C1-C8 alkyl, phenyl, or phenyl substituted by at least one group selected from halogen, cyano, nitro, C1-C8 alkyl, halo C1-C8 alkyl, C1-C8 alkoxycarbonyl, C1-C8 alkylthio, C1-C8 alkylsulfonyl, C1-C8 alkoxy, or halo C1-C8 alkoxy;
m represents 0, 1, or 2; n represents 0 or 1; s represents 0, 1, 2, 3, 4, or 5.

3. The oxabicycloalkane compound according to claim 1, wherein it is **characterized in that**,
A and B each independently represent C1-C6 alkyl;
X, Y, and Z each independently represent hydrogen, nitro, halogen, cyano, formyl, thiocyanato, sulfhydryl, OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C6 alkyl)-C(S)R¹, -(C1-C6 alkyl)-C(S)OR¹, -(C1-C6 alkyl)-C(O)SR¹, -(C1-C6 alkyl)-C(S)SR¹, -(C1-C6 alkyl)-SC(O)R¹, -(C1-C6 alkyl)-OC(S)R¹, -(C1-C6 alkyl)-SC(S)R¹, -O-(C1-C6 alkyl)-NR⁴R⁵, -S-(C1-C6 alkyl)-NR⁴R⁵, -(C1-C6 alkyl)-O-(C1-C6 alkyl)-NR⁴R⁵, -(C1-C6 alkyl)-S-(C1-C6 alkyl)-NR⁴R⁵, -(C1-C6 alkyl)-(C=S)ₙ-NR⁴R⁵, -NH-(C1-C6 alkyl)-NR⁴R⁵, -(C1-C6 alkyl)-OR¹, -(C1-C6 alkyl)-COR¹, -(C1-C6 alkyl)-CO₂R¹, -(C1-C6 alkyl)-OCOR¹, -(C1-C6 alkyl)-NR³COR¹, -(C1-C6 alkyl)-SO₂OR¹, -(C1-C6 alkyl)-NR³SO₂R², -(C1-C6 alkyl)-OSO₂R², -(C1-C6 alkyl)-S(O)ₘR², -(C1-C6 alkyl)-CONR⁴R⁵, -(C1-C6 alkyl)-SO₂NR⁴R⁵, NR⁴R⁵, 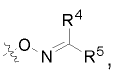 P(O)(OR⁶)₂, CH₂P(O)(OR⁶)₂, -(C1-C6 alkyl)-CN, C1-C6 alkyl, C2-C6 alkenyl, C2-C6 alkynyl, C3-C6 cycloalkyl, C3-C6 cycloalkyl C1-C6 alkyl, C3-C6 cycloalkenyl, C3-C6 cycloalkenyl C1-C6 alkyl, heterocyclyl, heterocyclyl C1-C6 alkyl, aryl, or aryl C1-C6 alkyl;
wherein, the "C1-C6 alkyl", "C2-C6 alkenyl" or "C2-C6 alkynyl" are each independently unsubstituted or substituted by halogen; the "C3-C6 cycloalkyl", "C3-C6 cycloalkyl C1-C6 alkyl", "C3-C6 cycloalkenyl", "C3-C6 cycloalkenyl C1-C6 alkyl", "heterocyclyl", "heterocyclyl C1-C6 alkyl", "aryl" or "aryl C1-C6 alkyl" are each independently unsubstituted or substituted by at least one group selected from oxo, halogen, cyano, nitro, C1-C6 alkyl, C2-C6 alkenyl, C2-C6 alkynyl, C3-C6 cycloalkyl, halo C1-C6 alkyl, halo C2-C6 alkenyl, halo C2-C6 alkynyl, halo C3-C6 cycloalkyl, C3-C6 cycloalkyl substituted by C1-C6 alkyl, -OR¹⁰, -SR¹⁰, -(CO)OR¹⁰, -(SO₂)R¹⁰, -N(R¹⁰)₂, or -O-(C1-C6 alkyl)-(CO)OR¹⁰, or two adjacent carbon atoms on the ring form a fused ring with -OCH₂CH₂- or -OCH₂O- that is unsubstituted or substituted by halogen;
R¹, R³, R⁴, and R⁵ each independently represent hydrogen, aryl, aryl C1-C6 alkyl, heterocyclyl, heterocyclyl C1-C6 alkyl, C1-C6 alkyl, halo C1-C6 alkyl, C2-C6 alkenyl, halo C2-C6 alkenyl, C2-C6 alkynyl, halo C2-C6 alkynyl, C3-C6 cycloalkyl, halo C3-C6 cycloalkyl, C1-C6 alkoxy C1-C6 alkyl, C3-C6 cycloalkyl C1-C6 alkyl; wherein the latter 10 groups are substituted by s substituents selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C6 alkoxy C1-C6 alkoxycarbonyl;
R² is aryl, aryl C1-C6 alkyl, heterocyclyl, heterocyclyl C1-C6 alkyl, C1-C6 alkyl, C2-C6 alkenyl, C2-C6 alkynyl, C3-C6 cycloalkyl, C3-C6 cycloalkyl C1-C6 alkyl; wherein the latter 5 groups are substituted by s groups selected from cyano, halogen, nitro, cyanothio, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C6 alkoxy C1-C6 alkoxycarbonyl;
R⁶ is methyl or ethyl;
R⁷ and R⁸ each independently represent hydrogen, C1-C6 alkyl, C2-C6 alkenyl, or C2-C6 alkynyl;
R⁹ is C1-C6 alkyl, C2-C6 alkenyl, or C2-C6 alkynyl;
R¹⁰ independently represents hydrogen, C1-C6 alkyl, halo C1-C6 alkyl, phenyl, or phenyl substituted by at least one group selected from halogen, cyano, nitro, C1-C6 alkyl, halo C1-C6 alkyl, C1-C6 alkoxycarbonyl, C1-C6 alkylthio, C1-C6 alkylsulfonyl, C1-C6 alkoxy, or halo C1-C6 alkoxy;
m represents 0, 1, or 2; n represents 0 or 1; s represents 0, 1, 2, 3, 4, or 5.

4. The oxabicycloalkane compound according to claim 1, wherein it is **characterized in that**, the compound is selected from any one of Table 1 and Tables A-H.

5. A preparation method of the oxabicycloalkane compound according to any one of claims 1 to 4, wherein it is **characterized in that**, it comprises the following steps:
subjecting the compound shown in the general formula II and the compound shown in the general formula III to substitution reaction to produce the compound of the general formula I; the chemical reaction equation is as follows: 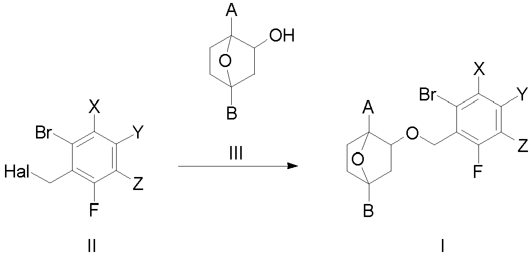
wherein, Hal represents halogen, and the definitions of other substituent groups A, B, X, Y, and Z are as shown in any one of claims 1 to 4;
preferably, the reaction is carried out in the presence of NaH or an anhydrous carbonate, and a solvent; more preferably, the solvent is selected from at least one of DMF, dimethylacetamide, THF, acetonitrile, dichloroethane, DMSO, dioxane, dichloromethane, or ethyl acetate.

6. A herbicidal composition, wherein it is **characterized in that**, it comprises (i) a herbicidally effective amount of at least one of the oxabicycloalkane compounds according to any one of claims 1 to 4.

7. The composition according to claim 6, wherein it is **characterized in that**, it further comprises (ii) a herbicidally effective amount of one or more additional herbicides and/or safeners; preferably, the additional herbicides are selected from bensulfuron-methyl, pyrazosulfuron-ethyl, penoxsulam, thifensulfuron-methyl, flumetsulam, 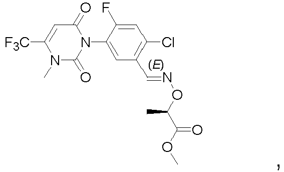 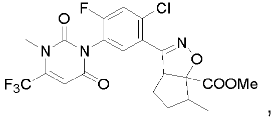 oxadiazon, oxadiargyl, oxyfluorfen, pyraclonil, flumioxazin, propanil, bentazone, isoproturon, chlorotoluron, metribuzin, atrazine, terbuthylazine, prometryn, 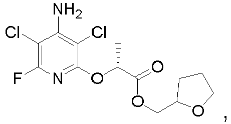 florpyrauxifen-benzyl, fluroxypyr, MCPA-isooctyl, 2,4-D-isooctyl, 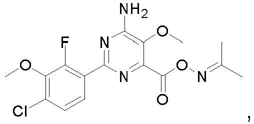 tripyrasulfone, 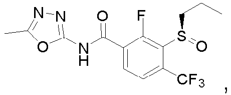 bipyrazone, fenpyrazone, mesotrione, benzobicyclon, tefuryltrione, isoxaflutole, acetochlor, pretilachlor, butachlor, mefenacet, fentrazamide, anilofos, pyroxasulfone, triallate, pendimethalin, butralin, diflufenican, beflubutamid-M, 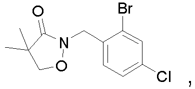 clomazone, bixlozone, oxaziclomefone, cyhalofop-butyl, metamifop, and profoxydim.

8. The composition according to any one of claims 6 to 7, wherein it is **characterized in that**, it further comprises (iii) an agrochemically acceptable formulation auxiliary.

9. A method for controlling undesirable plants, wherein it is **characterized in that**, it comprises applying a herbicidally effective amount of at least one of the oxabicycloalkane compounds according to any one of claims 1 to 4 or the herbicidal composition according to any one of claims 6 to 8 to a plant, or in its area, or to soil or water to control the emergence or growth of undesirable plants; preferably, the undesirable plants include herbicide-resistant or -tolerant weed species.

10. A use of at least one of the oxabicycloalkane compounds according to any one of claims 1 to 4 or the herbicidal composition according to any one of claims 6 to 8 for controlling undesirable plants; preferably, the oxabicycloalkane compound is used to control weeds among useful crops; more preferably, the useful crops include transgenic crops or crops treated by genome editing techniques, and the weeds include herbicide-resistant or -tolerant weed species.
